# EUROPEAN PATENT SPECIFICATION

(11) **EP 2 668 209 B1**
(45) Date of publication and mention of the grant of the patent: **30.12.2020**
(21) Application number: 12738907.0
(22) Date of filing: 20.01.2012
(51) Int. Cl.: C07K 16/28, A61K 39/395, A61P 35/00, C07K 16/46

(54) **ANTIBODIES SELECTIVE FOR CELLS PRESENTING EGFR AT HIGH DENSITY**
FÜR ZELLEN MIT EGFR BEI HOHER DICHTE SELEKTIVE ANTIKÖRPER
ANTICORPS SÉLECTIFS DES CELLULES PRÉSENTATRICES DES RÉCEPTEURS DE EGF À HAUTE DENSITÉ

(30) Priority: 24.01.2011 US 201161435510 P
(43) Date of publication of application: 04.12.2013
(73) Proprietor: YM Biosciences Inc., Mississauga, ON L4W 4Y5 (CA); National Research Council of Canada, Ottawa, Ontario K1A 0R6 (CA)
(72) Inventor: TIKHOMIROV, Ilia Alexandre, Toronto Ontario M8Z 1P5 (CA); JARAMILLO, Maria L., Beaconsfield Québec H9W 2M2 (CA); O'CONNOR-MCCOURT, Maureen D., Beaconsfield Québec H9W 1P2 (CA); SULEA, Traian, Kirkland Québec H9J 3A2 (CA); GILBERT, Renald, Montreal Québec H4V 2B4 (CA); GAILLET, Bruno, Sainte Julienne Québec J0K 2T0 (CA); BAARDSNES, Jason, Montreal Québec H3K 1N8 (CA); BANVILLE, Myriam, Laval Québec H7X 3K4 (CA); GROTHE, Suzanne, Montreal Québec H4H 1X8 (CA)
(74) Representative: Greaves Brewster LLP
(86) International application number: PCT/CA2012/050034
(87) International publication number: WO 2012/100346

(56) References cited:
- WO-A1-96/40210
- WO-A2-2008/104183
- Ilia A. Tikhomirov ET AL: "Binding Binding Properties of Epidermal Growth Factor Receptor (EGFR) Targeted Monoclonal Antibodies: Factors Contributing to Differences in Observed Clinical Profiles (A36)", AACR Cancer Clinical Trials and Personalized Medicine, 1 August 2008 (2008-08-01), XP055197559, Retrieved from the Internet: URL:http://ymbiosciences.q4web.com/files/d oc_downloads/Nimotuzumab/poster_nimo_AACR_ Translational_Med_2008_v001_q194wg.pdf [retrieved on 2015-06-23]
- MELARKODE RAMAKRISHNAN ET AL: "Nimotuzumab, a promising therapeutic monoclonal for treatment of tumors of epithelial origin", MABS, 1 January 2009 (2009-01-01), pages 41-48, XP055173443,
- A. TALAVERA ET AL: "Nimotuzumab, an Antitumor Antibody that Targets the Epidermal Growth Factor Receptor, Blocks Ligand Binding while Permitting the Active Receptor Conformation", CANCER RESEARCH, vol. 69, no. 14, 15 July 2009 (2009-07-15) , pages 5851-5859, XP055197598, ISSN: 0008-5472, DOI: 10.1158/0008-5472.CAN-08-4518
- ARLHEE DIAZ MIQUELI ET AL: "Biological Activity In Vitro of Anti-Epidermal Growth Factor Receptor Monoclonal Antibodies with Different Affinities", HYBRIDOMA, vol. 26, no. 6, 1 December 2007 (2007-12-01), pages 423-432, XP055197602, ISSN: 1554-0014, DOI: 10.1089/hyb.2007.0516
- CROMBET T ET AL: "Use of the Humanized Anti-Epidermal Growth Factor Receptor Monoclonal Antibody h-R3 in Combination with Advanced Head and Neck Cancer Patients", JOURNAL OF CLINICAL ONCOLOGY, AMERICAN SOCIETY OF CLINICAL ONCOLOGY, US, vol. 22, no. 9, 1 May 2004 (2004-05-01), pages 1646-1654, XP008131473, ISSN: 0732-183X, DOI: 10.1200/JCO.2004.03.089
- STEPHEN I. RUDNICK ET AL: "Affinity and Avidity in Antibody-Based Tumor Targeting", CANCER BIOTHERAPY & RADIOPHARMACEUTICALS, vol. 24, no. 2, 1 April 2009 (2009-04-01), pages 155-161, XP055069540, ISSN: 1084-9785, DOI: 10.1089/cbr.2009.0627
- MASAYUKI ODA ET AL: "Reevaluation of stoichiometry and affinity/avidity in interactions between anti-hapten antibodies and mono- or multi-valent antigens", MOLECULAR IMMUNOLOGY, vol. 37, no. 18, 1 December 2000 (2000-12-01), pages 1111-1122, XP055197715, ISSN: 0161-5890, DOI: 10.1016/S0161-5890(01)00028-1
- GRETA GARRIDO ET AL: "Bivalent binding by intermediate affinity of nimotuzumab: A contribution to explain antibody clinical profile", CANCER BIOLOGY & THERAPY, vol. 11, no. 4, 15 February 2011 (2011-02-15), pages 373-382, XP055197571, ISSN: 1538-4047, DOI: 10.4161/cbt.11.4.14097
- BOLAND, W ET AL.: 'The Emerging Role OfNimotuzumab In The Treatment Of Non-Small Cell Lung Cancer.' BIOLOGICS vol. 4, 09 November 2010, pages 289 - 298, XP055115351
- DONALDSON, J. M. ET AL.: 'Design And Development Of Masked Therapeutic Antibodies To Limit Off- Target Effects: Application To Anti-EGFR Antibodies.' CANCER BIOL THER vol. 8, November 2009, pages 2147 - 2152, XP009135774

## Description

### Field of the Invention

This invention relates to antibodies having therapeutic and diagnostic utility. More particularly, the present invention relates to antibodies that bind selectively to cells that present EGFR (epidermal growth factor receptor) at abnormally high density. The antibodies are useful therapeutically and diagnostically in the fields of oncology and other diseases.

### Background to the Invention

Drugs for the treatment of cancer and other diseases have a so-called "therapeutic window". In the case of cancer, the therapeutic window defines the drug dosage that can kill cancer cells preferentially to normal cells, thereby establishing a safety range for the use of the drug. The therapeutic window for conventional chemotherapeutics is narrow with, in many cases, significant adverse effects coinciding with marginal slowing of tumour growth. Targeted treatments that spare normal cells are urgently needed.

Therapeutic antibodies form a newer class of cancer therapies that specifically target an antigen presented on the surface of cancer cells. When the target surface protein is unique to the cancer cell, adverse antibody effects on normal cells can be avoided. However, for the majority of antigens, target expression is not restricted completely to tumour cells, with some normal cells also expressing the antigen. In these cases, the antibody may have an effect on normal cells as well as tumor cells, leading to "on-target, off-tissue" adverse events. In the case of the EGFR antigen, because of its ubiquitous presence on the surface of normal cells such as keratinocytes as well as on cancer cells, the clinical use of EGFR-targeting therapeutics is associated with adverse events that include severe rash.

Considering the efficacy of anti-EGFR therapies in treating patients that overexpress EGFR, the risk associated with severe skin reaction is currently considered acceptable when managed properly. The risk of anti-EGFR therapy-associated toxicity can be reduced by prior administration of anti-histamine, or by administering anti-EGFR antibody at a reduced and less effective dose.

Efforts to improve upon EGFR antibodies are aimed at generating antibodies having even greater affinity for the target antigen. In WO 2006/009694 published 26 January 2006, Kussie et al describe the crystal structure of the interaction between EGFR and cetuximab Fab fragment, and identify residues that may be modified to improve the effectiveness of cetuximab as an EGFR antagonist.

Tikhomirov et al., (Cancer Clinical Trails and Personalized Medicine, (2008-08-01), XP055197559) describes nimotuzumab, an EGFR antibody that binds preferentially to tumor cells that express moderate to high levels of EGFR over cells having low density of EGFR.

It would be desirable to provide an EGFR antibody that is useful to treat subjects presenting with EGFR over-expressing disease cells, while avoiding significant interaction with tissues including skin and particularly keratinocytes and other cells that also present the EGFR antigen at normal levels.

It is an object of the present invention to provide therapeutic antibodies, and fragments and conjugates thereof that bind effectively to a given target only when that target is presented at a relatively higher density characteristic of a disease state.

It is a further object of the present invention to provide such antibodies, fragments and conjugates in pharmaceutical compositions, particularly for therapeutic and diagnostic use.

It is a further object of the present invention to provide a method useful, in a subject in need thereof, to control the growth of disease cells that present EGFR at a density greater than normal EGFR density, while avoiding or minimizing adverse effects on normal cells.

### Summary of the Invention

In one aspect, the present invention provides an isolated, EGFR antibody or bivalent fragment thereof that binds preferentially to target cells that present EGFR at a density above a normal EGFR density. Cells that present EGFR at a density greater than normal EGFR density are disease cells, including cancer cells such as colorectal and other cancer cells, that over-express the EGFR gene, and manifest on their surface a greater number of EGFR proteins than cells that express the EGFR gene at normal levels.

The antibodies of the present invention, and their bivalent fragments, display a preference for binding to disease cells having the higher EGFR density, and show reduced and desirably minimal or negligible, i.e., insignificant, binding to normal cells having a normal EGFR density. The present antibodies and their bivalent binding fragments thus are well suited for use in reducing or eradicating high density EGFR disease cells while minimizing or avoiding effects on normal cells, thereby reducing the number or severity of adverse events in subjects receiving EGFR antibody therapy.

The EGFR antibody of the invention comprises a heavy chain and a light chain, each chain having a constant region and a variable region, each variable region comprising framework regions and complementarity determining regions (CDRs), wherein the CDRs have an amino acid sequence set forth below:

**For the heavy chain:**

| | | |
|---|---|---|
| CDR1 | NYGVH | (SEQ ID No. 1) |
| CDR2 | VIWSGGNTD⁵⁸YNTPFTS | (SEQ ID No. 2) |
| CDR3 | ALTY¹⁰¹Y¹⁰²D¹⁰³YE¹⁰⁵FAY | (SEQ ID No. 3) |

**For the light chain:**

| | | |
|---|---|---|
| CDR1 | RASQSIGTNIH | (SEQ ID No. 4) |
| CDR2 | ASE⁵³ SIS | (SEQ ID No. 5) |
| CDR3 | QQNNNW⁹⁴PTT | (SEQ ID No. 6) |

| | | |
|---|---|---|
| wherein at least one of E⁵³ is substituted by K⁵³, D⁵⁸ is substituted by N⁵⁸, W⁹⁴ is substituted by A⁹⁴, Y¹⁰¹ is substituted by A¹⁰¹, Y¹⁰² is substituted by A¹⁰², D¹⁰³ is substituted by N¹⁰³, and E¹⁰⁵ is substituted by Q¹⁰⁵, wherein the substituted amino acid confers on said antibody a reduced EGFR binding affinity (Kd) that is 1.0 nM or weaker, wherein when D⁵⁸ is substituted by N⁵⁸ this is in combination with D¹⁰³ being substituted by N¹⁰³ and when E¹⁰⁵ is substituted by Q¹⁰⁵ this is in combination with D⁵⁸ being substituted by N⁵⁸ and D¹⁰³ being substituted by N¹⁰³. | | |

In embodiments, the substituting amino acid(s) are selected to confer on the antibody a binding affinity (Kd) for EGFR that is about 10 fold or more weaker than the EGFR binding affinity of cetuximab.

In embodiments, the present invention provides an EGFR antibody comprising a heavy chain and a light chain, each chain having a constant region and a variable region, wherein the heavy chain variable region comprises the sequence of SEQ ID No. 8 and the light chain variable region comprises the sequence of SEQ ID No. 7, wherein at least one of E⁵³ is substituted by K⁵³, D⁵⁸ is substituted by N⁵⁸, W⁹⁴ is substituted by A⁹⁴, Y¹⁰¹ is substituted by A¹⁰¹, Y¹⁰² is substituted by A¹⁰², D¹⁰³ is substituted by N¹⁰³, and E¹⁰⁵ is substituted by Q¹⁰⁵, wherein the substituted amino acid confers on said antibody a reduced EGFR binding affinity (Kd) that is 1.0 nM or weaker, wherein when D⁵⁸ is substituted by N⁵⁸ this is in combination with D¹⁰³ being substituted by N¹⁰³ and when E¹⁰⁵ is substituted by Q¹⁰⁵ this is in combination with D⁵⁸ being substituted by N⁵⁸ and D¹⁰³ being substituted by N¹⁰³.

In other embodiments, the substituting amino acid is selected to reduce EGFR binding affinity of the antibody or bivalent fragment to a level that substantially eliminates binding to cells presenting EGFR at a normal EGFR density, and retains effective binding at targeted disease cells that present EGFR at a greater density relative to normal cell EGFR density.

In still other embodiments, the antibody or bivalent fragment is a variant of cetuximab having one or more substitutions at the residues identified herein. In particular embodiments, the substitutions are non-conservative amino acid substitutions.

In another of its aspects, the present invention provides conjugates, i.e., immunoconjugates, comprising an antibody or bivalent fragment thereof according to the present invention and, conjugated therewith, an agent useful to treat or detect cells presenting EGFR at a density characteristic of disease cells.

In a further aspect, the present invention provides medically useful compositions comprising an antibody, bivalent fragment thereof or immunoconjugate thereof according to the present invention, in combination with a medically acceptable carrier, such as a pharmaceutically acceptable carrier or a diagnostically useful carrier.

In a related aspect, the present invention provides a method for treating a subject having disease cells that present EGFR at a density greater than the EGFR density on normal cells, comprising the step of administering to the subject an effective amount of an antibody, bivalent fragment thereof, or an immunoconjugate of the present invention. Subjects so treated will manifest adverse events that are fewer in number and/or severity given the reduced affinity of the present antibodies for normal cells and tissue.

These and other aspects of the present invention are now described in greater detail with reference to the accompanying drawings, in which:

### Reference to the Figures

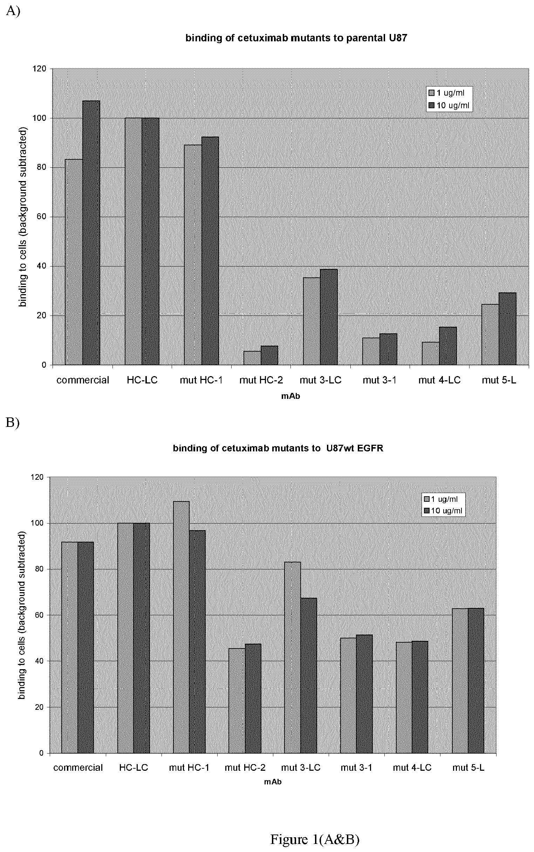

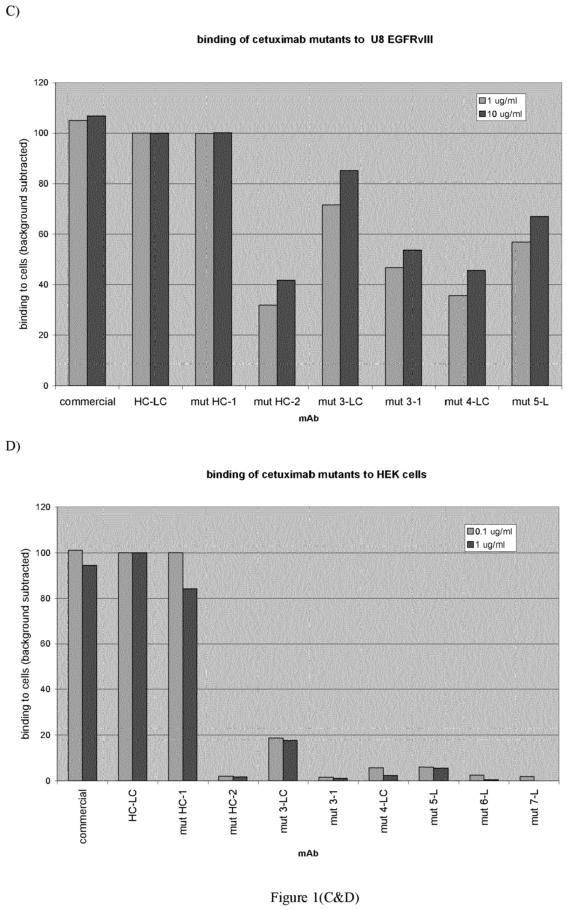

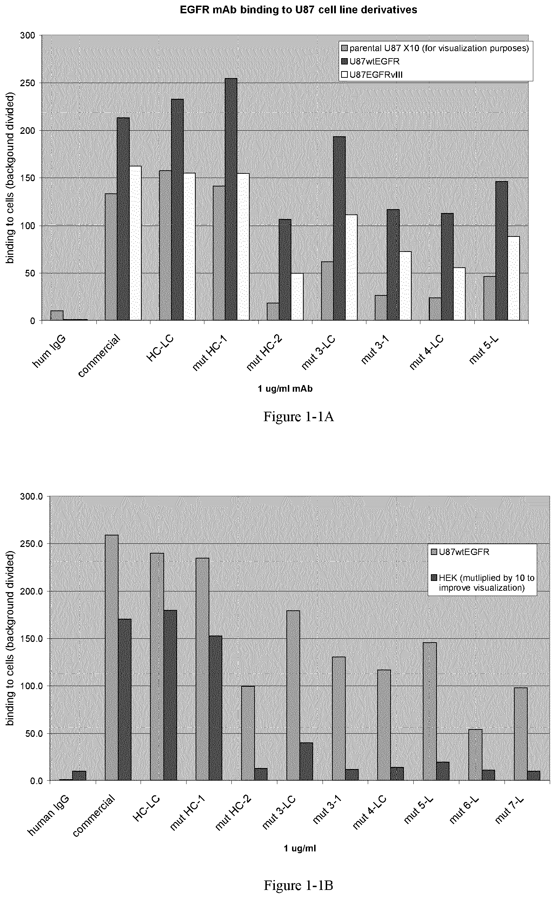

Figure 1 is a graph showing binding of antibodies to cell surface EGFR present on the surface of (A) parental U87MG cells, (B) U87MGwtEGFR, U87 cells engineered to overexpress wild type (wt) EGFR, (C) U87MG-EGFRvIII, U87 cells engineered to overexpress EGFR-vIII and (D) primary human epidermal keratinocytes (HEK), at 1 and 10 µg/ml mAb (A-C) or 0.1 and 1 µg/ml mAb (D). These can be compared to wild type (wt)-mAb (HC/LC) which was set arbitrarily to 100%. Similarly, Figure 1-1 (A-B) shows results from these same experiments plus additional experiments, but using a different data presentation approach, i.e. all binding is divided by background binding, (that is, is expressed as a fold change over background binding) rather than background binding being subtracted from all binding values (as was done in Figure 1). These results demonstrate a greater reduction in binding of some anti-EGFR mAb variants to cells expressing lower EGFR levels (parental U87 or HEK cells) as compared to the reduction observed on U87 cells overexpressing EGFR.

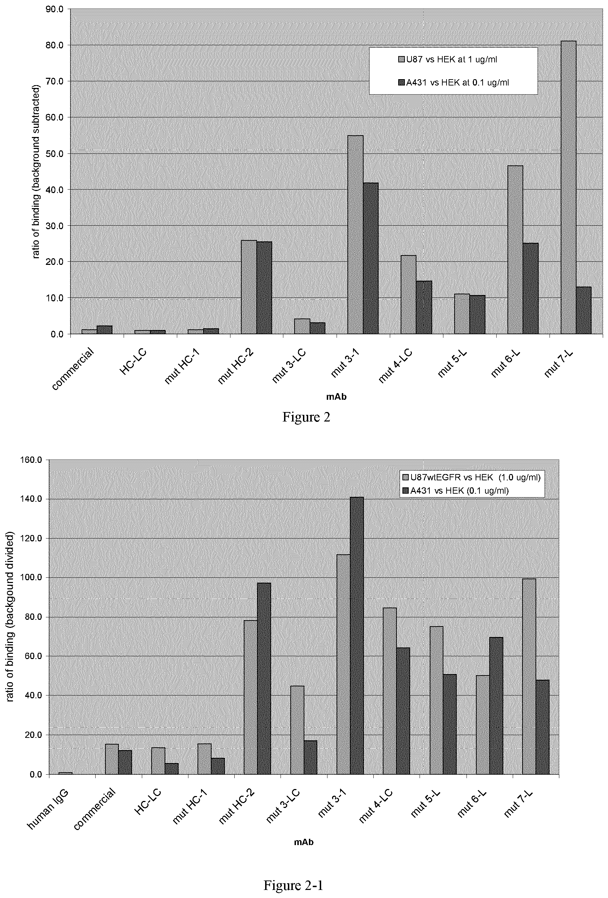

Figure 2 is a graph representing binding selectivity of antibodies. The ratio of antibody binding (with background subtracted) to EGFR overexpressing cells [U87MGwtEGFR or A431 cells (which naturally overexpress wt EGFR)] relative to antibody binding to normal HEK cells was calculated and compared to that seen with wild type antibody (ratio set arbitrarily to 1 for wt antibody). In Figure 2-1, the same results as in Figure 2 are shown using a different data presentation approach, i.e. all binding is divided by background. Note - all additional figures present data analysed in this manner. These results clearly show that some of the EGFR mAbs exhibit better binding to tumor cells that overexpress EGFR relative to normal HEK cells (e.g. mutant HC-2 exhibits a 20-fold (Fig. 2) or 6-fold (Fig. 2-1) better ratio of binding, and mutant 3-1 exhibits a 40-fold (Fig. 2) or 9-fold (Fig. 2-1) better ratio of binding to tumor than normal cells). The pattern of binding specificity was similar amongst the tumor cell lines analyzed (U87MGwt EGFR and A431) suggesting that the selectivity of binding is universally high for tumor cells overexpressing EGFR (~2 million receptors per cell or more).

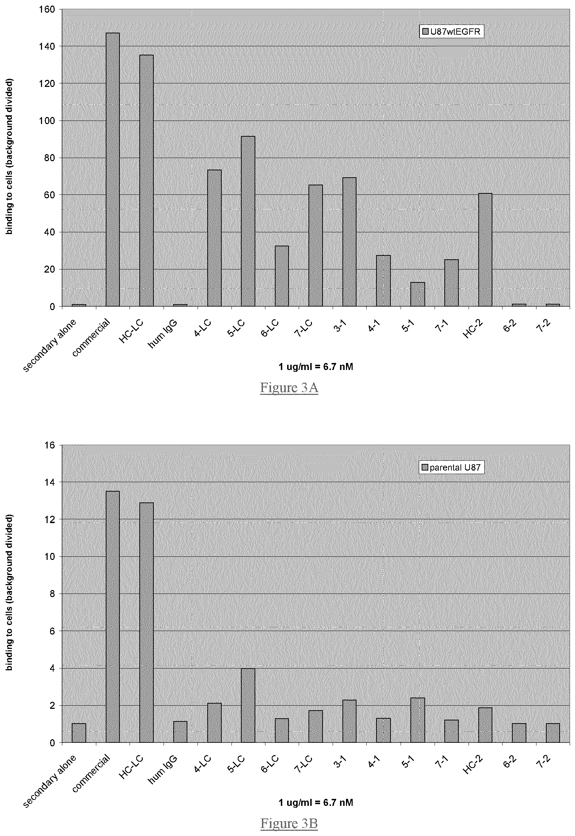

Figure 3 depicts graphs showing binding of mutated antibodies at 1µg/ml (6.7 nM) to (A) U87MGwtEGFR, U87 cells overexpressing wt EGFR, and (B) parental U87MG cells.

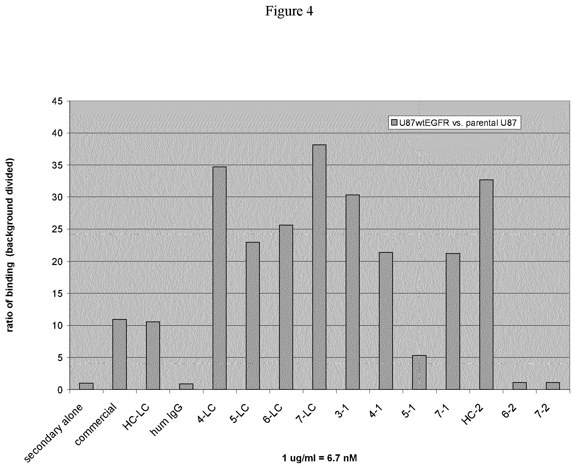

Figure 4 is a graph illustrating the binding selectivity of antibodies, based on data from Figure 3. The ratio of antibody binding to EGFR overexpressing cells (U87MGwtEGFR) relative to antibody binding to parental U87MG cells was calculated. This results in a ratio of 11 for wild type antibody binding to U87MGwtEGFR cells versus parental cells; and in ratios of up to 35 for certain mutated antibodies, e.g. mutant 7-LC and 4-LC. In other words, these mutant antibodies show a 3-4 fold better ratio (selectivity) of binding.

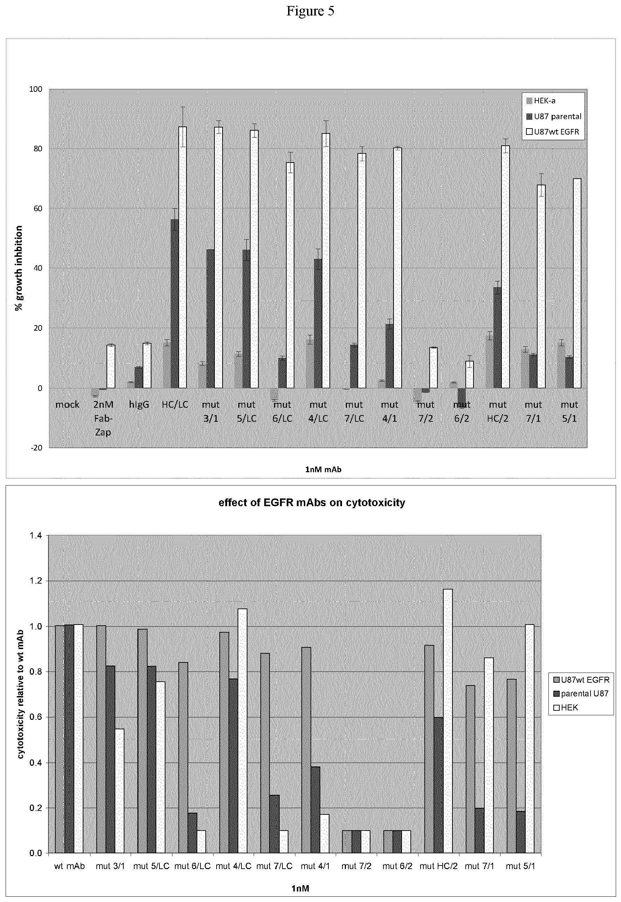

Figure 5 illustrates the ability of the EGFR mAbs to bind cells and deliver a protein toxin, saporin. Specifically, 1 nM EGFR mAbs were incubated with 2 nM anti-human secondary antibody that was chemically conjugated with saporin toxin (Advanced Targeting Systems, San Diego, CA), a ribosome inactivating enzyme that needs to be internalized to cause cell death. The antibody complex was then added to the cell types indicated (plated in triplicate) and their effects on cell viability were measured after 72 h incubation at 37°C. EGFR directed cytotoxicity can be quantitated following evaluation with controls for non-specific cytotoxicity [in which no primary or an irrelevant primary antibody (control human IgG) were used].

### Detailed Description of the Invention and Preferred Embodiments

As used herein, the term "EGFR" refers to any protein that comprises the expressed and processed product of the her-1 gene, wherein the protein is designated as UniProtKB/Swiss-Prot P04626-1, including antibody-binding variants thereof.

The present invention relates to EGFR antibodies and bivalent fragments thereof that display a preference for binding to disease cells presenting EGFR at a density greater than normal cells. On cells that present EGFR, the normal density of EGFR is generally less than about 10,000 EGFR molecules per cell, and is usually less than about 1,000 EGFR molecules per cell. EGFR-presenting disease cells, on the other hand, present EGFR at a density generally greater than 10,000 EGFR molecules per cell, and usually greater than about 100,000 EGFR molecules per cell. Generally, the EGFR density is thus about 10³ or less on normal cells, and about 10⁵ or more on disease cells. The actual number of EGFR molecules on any given cell can be determined by established methods, including the antibody based radiolabeled binding or flow cytometry binding to live cells herein exemplified. The binding avidity of the present antibodies is greater for the higher EGFR density disease cells than for the lower EGFR density normal cells. This greater avidity is revealed conveniently using techniques established for determining affinity constants for antibody-target interactions, also as exemplified herein.

In embodiments, the present EGFR antibodies having a binding affinity for EGFR that is about 10-fold or more weaker than the EGFR binding affinity of cetuximab. Desirably, the binding affinity of the antibody for EGFR is about 15-fold, 20-fold, 25-fold, and preferably 30-fold or more weaker than the EGFR binding affinity of cetuximab. In absolute terms, and given an EGFR binding affinity of about 0.3 nM for cetuximab, the present antibodies incorporate amino acid substitution(s) that reduce their EGFR binding affinity (Kd) to about 1.0 nM and weaker, more desirably about 10 nM and weaker, e.g., to an EGFR binding affinity that is in the range from 1 nM to 1 µM, more desirably 2 nM to 500 nM, such as 10 nM to 500 nM or 10 nM to 100 nM.

In embodiments, the antibody is an intact antibody comprising features common to all natural antibodies, and thus comprises a heavy chain and a light chain, each chain having a constant region and a variable region, each variable region comprising framework regions (FRs) and complementarity determining regions (CDRs). In the alternative, the antibody is provided as a bivalent fragment, i.e., an antibody fragment comprising both "arms" of an intact antibody, joined through a linker that can be represented by the hinge region of the antibody or any equivalent. Such bivalent fragments include F(ab)₂ fragments and any other bivalent fragment that retains preference for high density EGFR. In particular embodiments, the bivalent fragment is a F(ab')₂ fragment, generated for instance by papain-based digestion of the parent antibody using standard procedures for digestion and subsequent fragment isolation. In the alternative, the bivalent fragment can be a so-called single chain Fv (scFv), consisting of the variable light and variable heavy antibody domains joined by an amino acid linker, or a bivalent form of a so-called diabody prepared using a 5 amino acid linker such as SGGGG between the light and heavy chain variable domains and a C-terminal cysteine modification to GGC to give a final diabody product as VL-SGGG-VH-GGC. Still other bivalent fragments can be prepared by coupling the light and heavy chain variable domains through thioether linkages such as bis-maleimidomethyl ether (BMME), N,N' -p-phenylene dimaleimide (PDM and N,N'-bismaleimidohexane BMH), to stabilize the F(ab')2 fragments.

In the intact antibody or bivalent fragment, the CDRs comprise or consist of the following amino acid sequences:

**For the heavy chain:**

| | | |
|---|---|---|
| CDR1 | NYGVH | (SEQ ID No. 1) |
| CDR2 | VIWSGGNTD⁵⁸YNTPFTS | (SEQ ID No. 2) |
| CDR3 | ALTY¹⁰¹Y¹⁰²D¹⁰³YE¹⁰⁵FAY | (SEQ ID No. 3) |

**For the light chain:**

| | | |
|---|---|---|
| CDR1 | RASQSIGTNIH | (SEQ ID No. 4) |
| CDR2 | ASE⁵³ SIS | (SEQ ID No. 5) |
| CDR3 | QQNNNW⁹⁴PTT | (SEQ ID No. 6) |

| | | |
|---|---|---|
| wherein at least one of E⁵³ is substituted by K⁵³, D⁵⁸ is substituted by N⁵⁸, W⁹⁴ is substituted by A⁹⁴, Y¹⁰¹ is substituted by A¹⁰¹, Y¹⁰² is substituted by A¹⁰², D¹⁰³ is substituted by N¹⁰³, and E¹⁰⁵ is substituted by Q¹⁰⁵, wherein the substituted amino acid confers on said antibody a reduced EGFR binding affinity (Kd) that is 1.0 nM or weaker, wherein when D⁵⁸ is substituted by N⁵⁸ this is in combination with D¹⁰³ being substituted by N¹⁰³ and when E¹⁰⁵ is substituted by Q¹⁰⁵ this is in combination with D⁵⁸ being substituted by N⁵⁸ and D¹⁰³ being substituted by N¹⁰³. | | |

The substituting amino acids are most suitably genetically encoded amino acids that are selected desirably, but not essentially, from an amino acid class that is different from the amino acid class to which the parent amino acid belongs. For instance, in the case of Y¹⁰¹ and Y¹⁰², suitable substituting amino acids are those that are not polar/neutral/large amino acids. The selection process can be conducted by applying computer aided tools that couple saturation virtual mutagenesis engines with algorithms for *in silico* scoring of binding affinities and/or association rates. Amino acid selections can also be made based on the following Table 1:

| Amino Acid Size* | 3-letter | | 1-letter (side chain) | Polarity (pH 7.4) | Charge |
|---|---|---|---|---|---|
| Alanine | Ala | A | nonpolar | neutral | tiny |
| Arginine | Arg | R | polar | positive | large |
| Asparagine | Asn | N | polar | neutral | small |
| Aspartic acid | Asp | D | polar | negative | small |
| Cysteine | Cys | C | nonpolar | neutral | small |
| Glutamic acid | Glu | E | polar | negative | small |
| Glutamine | Gln | Q | polar | neutral | small |
| Glycine | Gly | G | nonpolar | neutral | tiny |
| Histidine | His | H | polar | neutral (90%) | large |
| Isoleucine | Ile | I | nonpolar | neutral | large |
| Leucine | Leu | L | nonpolar | neutral | large |
| Lysine | Lys | K | polar | positive | large |
| Methionine | Met | M | nonpolar | neutral | large |
| Phenylalanine | Phe | F | nonpolar | neutral | large |
| Proline | Pro | P | non-polar | neutral | small |
| Serine | Ser | S | polar | neutral | tiny |
| Threonine | Thr | T | polar | neutral | small |
| Tryptophan | Trp | W | nonpolar | neutral | bulky |
| Tyrosine | Tyr | Y | polar | neutral | large |
| Valine | Val | V | nonpolar | neutral | small |

| | | | | | |
|---|---|---|---|---|---|
| * based on volume in A³, where 50-100 is tiny, 100-150 is small, 150-200 is large and >200 is bulky | | | | | |

It will be appreciated that the conservative amino acid families include (i) G, A, V, L and I; (ii) D and E; (iii) A, S and T; (iv) H, K and R; (v) N and Q; and (vi) F, Y and W.

In embodiments, the heavy chain variable region of the antibody or bivalent fragment incorporates at least one substitution at D⁵⁸, Y¹⁰¹, Y¹⁰², D¹⁰³, or E¹⁰⁵. In other embodiments, the heavy chain variable region incorporates substitutions at least two such residues, such as at D⁵⁸ and D¹⁰³, or three such residues, such as at D⁵⁸, D¹⁰³ and E¹⁰⁵. In an alternative embodiment, the heavy chain variable region is wild type and incorporates no such substitutions, provided there is at least one substitution in the light chain variable region.

In embodiments, in the heavy chain CDRs, Y¹⁰¹ and/or Y¹⁰², independently, is replaced by a substituting amino acid having a side chain that is nonpolar and/or a side chain that is non-neutral and/or a side chain that is not large. Desirably, Y¹⁰¹ and/or Y¹⁰² is replaced by an amino acid selected independently from A, C, G, I, L, M, F, W and V; preferably from A, G, I, L and V; and more preferably from A, V, I and L. In a specific embodiment, the tyrosine occurring at one or both of positions 101 and 102 is replaced by alanine, thus yielding the substitutions designated Y¹⁰¹A and Y¹⁰²A.

In other embodiments, D⁵⁸ in the heavy chain CDR2 and/or D103 in the heavy chain CDR3 is replaced, independently, by a substituting amino acid having a side chain that is nonpolar and/or is charge neutral or positive and/or is not small. Desirably, D⁵⁸ and/or D¹⁰³ is replaced by an amino acid having a side chain that is charge neutral or positive, as well as polar, as well as small, and is selected desirably from N and Q. In a specific embodiment, D⁵⁸ is replaced by N⁵⁸, thus yielding the substitution designated D⁵⁸N. In another specific embodiment D¹⁰³ is replaced by N¹⁰³, thus yielding the substitution designated D¹⁰³N.

In other embodiments, E¹⁰⁵ in the heavy chain CDR3 is replaced by a substituting amino acid having a side chain that is nonpolar and/or is charge neutral or positive and/or is not small. Desirably, E¹⁰⁵ is replaced by an amino acid having a side chain that is charge neutral or positive, as well as polar, as well as small, and is selected desirably from N and Q. In a specific embodiment, E¹⁰⁵ is replaced by Q¹⁰⁵, thus yielding the substitution designated E¹⁰⁵Q.

In embodiments, the light chain variable region of the antibody or bivalent fragment incorporates at least one substitution at E⁵³ or at W⁹⁴. In a specific embodiment, the light chain variable region comprises substitutions at both E⁵³ or at W⁹⁴. In another specific embodiment, the light chain variable region incorporates substitution only at E⁵³, or only at W⁹⁴. In an alternative embodiment, the light chain variable region is wild type and incorporates no such substitutions, provided there is at least one substitution in the heavy chain variable region.

When substituted, E⁵³ is replaced by a substituting amino acid having a side chain that is either nonpolar and/or is neutral or positive in charge and/or may not be small. In embodiments, E⁵³ is substituted by an amino acid selected from R, D, E, H, or K. In a preferred embodiment, E⁵³ is substituted by K, yielding the substitution designated E⁵³K.

When substituted, W⁹⁴ is replaced by a substituting amino acid having a side chain that is either polar and/or is charge positive or negative and/or is not bulky. In embodiments, W⁹⁴ is replaced by R, N, D, E, Q, H, K, A, S, T or Y. In particular embodiments, W⁹⁴ is replaced by N, Q, H, S, T, A or Y. In a preferred embodiment, W⁹⁴ is replaced by A, yielding the substitution designated W⁹⁴A.

The antibody or bivalent fragment thereof comprises at least one substitution at a location noted above. The at least one substitution can occur in either the light chain variable region or the heavy chain variable region. In specific embodiments, antibodies comprising single site substitutions include:
An antibody comprising an E⁵³K substitution in CDR2 of the light chain, wherein the CDRs are otherwise the wild type versions specified above; or wherein the light chain is otherwise the wild type version as set out in SEQ ID No. 7, or wherein the antibody is otherwise cetuximab, i.e. [E⁵³K]cetuximab.

An antibody comprising a W⁹⁴A substitution in CDR3 of the light chain, wherein the CDRs are otherwise the wild type versions specified above; or wherein the light chain is otherwise the wild type version as set out in SEQ ID No. 7, or wherein the antibody is otherwise cetuximab, i.e., [W⁹⁴A]cetuximab.

An antibody comprising a D⁵⁸N substitution in CDR2 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [D⁵⁸N]cetuximab

An antibody comprising a Y¹⁰¹A substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [Y¹⁰¹A]cetuximab.

An antibody comprising a Y¹⁰²A substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [Y¹⁰²A]cetuximab.

An antibody comprising a D¹⁰³N substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [D¹⁰³N]cetuximab.

An antibody comprising an E¹⁰⁵Q substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [E¹⁰⁵Q]cetuximab.

In other embodiments, the antibody or binding fragment thereof comprises at least two such substitutions, either in the light chain variable region, in the heavy chain variable region, or at least one substitution in each of the light and heavy chain variable regions. In specific embodiments, antibodies including at least two such substitutions include:

An antibody comprising both a E⁵³K substitution in CDR2 of the light chain and a Y¹⁰¹A substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [E⁵³K, Y¹⁰¹ A]cetuximab.

An antibody comprising both a E53K substitution in CDR2 of the light chain and a Y102A substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [E53K, Y102A]cetuximab.

An antibody comprising both a D⁵⁸N substitution in CDR2 of the heavy chain, and a D¹⁰³N substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [D⁵⁸N, D¹⁰³N]cetuximab.

An antibody comprising at least three substitutions, including a D⁵⁸N substitution in CDR2 of the heavy chain, a D¹⁰³N substitution in CDR3 of the heavy chain, and an E¹⁰⁵Q substitution in CDR3 of the heavy chain, wherein the CDRs are otherwise the wild type versions specified above; or wherein the heavy chain is otherwise the wild type version as set out in SEQ ID No. 8, or wherein the antibody is otherwise cetuximab, i.e., [D⁵⁸N, D¹⁰³N, E¹⁰⁵Q]cetuximab.

In preferred embodiments, the antibody is one of [E53K, Y102A]cetuximab, [D58N, D103N]cetuximab, or [D58N, D103N, E105Q]cetuximab.

In addition to the recited three CDRs present in each of the light and heavy chain variable regions, the heavy and light chains of the intact antibody comprise four intervening framework regions that present the CDRs in a conformation suitable for EGFR binding, and constant regions that confer antibody effector function. The CDRs can be integrated into any suitable acceptor antibody, by grafting the present CDRs into the acceptor antibody, in accordance with practices and techniques well established for the production of chimeric, humanized and human antibodies.

Particularly suitable acceptor antibodies are antibodies already known to have EGFR binding affinity. Such donor antibodies are most desirably of human origin, but they can also derive from acceptor antibodies of non-human origin, including mouse, rat, rabbit, goat, sheep, primate and the like. It will be appreciated that human antibody acceptor sequences different from those exemplified herein can be identified and used to accommodate the presently desired CDRs. This is achieved by modeling the structure of a preferred antibody using for instance the Swiss-Model [http://swissmodel.expasy.org/repository] or similar software and selecting, from among the numerous human antibody sequences available in public databases, a human acceptor antibody sequence that, with CDR sequences altered as herein preferred, approximates the same structural conformation as the preferred antibodies. In embodiments, the acceptor antibodies, and the resulting present antibodies, are of the IgG1 isotype, but they may also be IgG2 or IgG4. Moreover, the isotype of the antibody, as dictated by the constant region, can be manipulated to alter or eliminate the effector function of the resulting antibody. That is, the constant region of the present antibodies is either wild type human antibody constant region, or a variant thereof that incorporates amino acid modifications, i.e., amino acid additions, substitutions or deletions that alter the effector function of the constant region, such as to enhance serum half-life, reduce complement fixation, reduce antigen-dependent cellular cytotoxicity and improve antibody stability. The number of amino acid modifications in the constant region is usually not more than 20, such as 1-10 e.g., 1-5 modifications, including conservative amino acid substitutions.

In embodiments, the half-life of the antibody is improved by incorporating one more amino acid modification, usually in the form of amino acid substitutions, for instance at residue 252, e.g., to introduce Thr, at residue 254, e.g., to introduce Ser, and/or at residue 256 e.g., to introduce Phe. Still other modifications can be made to improve half-life, such as by altering the CHI or CL region to introduce a salvage receptor motif, such as that found in the two loops of a CH2 domain of an Fc region of an IgG. Such alterations are described for instance in US 5869046 and US 6121022.

Altered C1q binding, or reduced complement dependent cytotoxicity, can be introduced by altering constant region amino acids at locations 329, 331 and 322, as described in US 6194551. The ability of the antibody to fix complement can further be altered by introducing substitutions at positions 231 and 239 of the constant region, as described in WO94/029351.

The framework regions of the light and heavy chains of the present antibodies and fragments also desirably have the sequence of a human antibody variable region, but incorporating the CDRs herein specified. In embodiments, the heavy chain variable region is human IgG4 in origin. In specific embodiments, the heavy chain variable region is that of human IgG, such as the human IgG1 antibody variant having the sequence designated Genbank gi 2414502. Alternatively, and preferably, the heavy chain variable region is that of human IgG4 antibody species designated Genbank gi 2414502.

The framework regions of the heavy and light chains of the present antibodies may also incorporate amino acid modifications, i.e., amino acid deletions, additions or substitutions, which further improve upon the properties of the antibody or fragment, in accordance with techniques established for antibody humanization. Such framework modifications can be modeled on the framework regions of antibody sequences provided in public databases, and on framework regions of antibodies known to bind EGFR, such as those antibodies referenced in the background section hereof. Preferred framework substitutions are those which yield antibodies having a greater preference for binding EGFR at the higher density associated with disease cells, relative to normal cells.

Framework modifications can also be made to reduce immunogenicity of the antibody or to reduce or remove T cell epitopes that reside therein, as described for instance by Carr et al in US2003/0153043.

In accordance with embodiments of the present invention, the heavy and light chain variable regions are modeled on the antibody cetuximab, and comprise a heavy chain variable region of SEQ ID No. 8, and/or a light chain variable region having SEQ ID No.7, as follows:
Light chain variable region (VL):
   DILLTQSPVILSVSPGERVSFSCRASQSIGTNIHWYQQRTNGSPRLLIKYAS**E**⁵³SISGIPSRFSGSGSGTD FTLSINSVESEDIADYYCQQNNN**W⁹⁴**PTTFGAGTKLELK [SEQ ID No. 7]; wherein E⁵³ or W⁹⁴ are as defined hereinabove;
Heavy chain variable region (VH):
   QVQLKQSGPGLVQPSQSLSITCTVSGFSLTNYGVHWVRQSPGKGLEWLGVIWSGGNTD⁵⁸YNTPFTSRLSIN KDNSKSQVFFKMNSLQSNDTAIYYCARALT**Y**¹⁰¹**Y**¹⁰²**D**¹⁰³Y**E**¹⁰⁵FAYWGQGTLVTVSA [SEQ ID No. 8] ; wherein D⁵⁸, Y¹⁰¹, Y¹⁰², D¹⁰³, or E¹⁰⁵ are as defined hereinabove.

In more specific and preferred embodiments, the entire light and heavy chains of the intact antibody are set out below as SEQ ID Nos. 9 and 10, respectively:
Entire Light chain:
   DILLTQSPVILSVSPGERVSFSCRASQSIGTNIHWYQQRTNGSPRLLIKYAS**E⁵³**SISGIPSRFSGSGSGTD FTLSINSVESEDIADYYCQQNNN**W⁹⁴**PTTFGAGTKLELKRTVAAPSVFIFPPSDEQLKSGTASWCLLNNFY PREAKVQWKVDNALQSGNSQESVTEQDSKDSTYSLSSTLTLSKADYEKHKVYACEVTHQGLSSPVTKSFNRG E [SEQ ID No. 9]; wherein E⁵³ and W⁹⁴ are as defined hereinabove;
Entire Heavy chain:
   QVQLKQSGPGLVQPSQSLSITCTVSGFSLTNYGVHWVRQSPGKGLEWLGVIWSGGNT**D**⁵⁸YNTPFTSRLSIN KDNSKSQVFFKMNSLQSNDTAIYYCARALT**Y**¹⁰¹**Y**¹⁰²**D**¹⁰³Y**E**¹⁰⁵FAYWGQGTLVTVSAASTKGPSVFPLAPSS KSTSGGTAALGCLVKDYFPEPVTVSWNSGALTSGVHTFPAVLQSSGLYSLSSWTVPSSSLGTQTYICNVNH KPSNTKVDKRVEPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVLDSDGSFFLYSKLTVDKSRWQQG NVFSCSVMHEALHNHYTQKSLSLSPGK [SEQ ID No. 10]; whereinD⁵⁸, Y¹⁰¹, Y¹⁰², D¹⁰³, or E¹⁰⁵ are as defined hereinabove.

As noted, final selection of an antibody or binding fragment is made based on the binding preference displayed by the desired antibody or bivalent fragment for cells that present EGFR at a density greater than normal. The target cells are thus disease cells presenting greater than normal EGFR density, as a hallmark. Screening can be performed *in vitro,* as exemplified herein, using as reference cells a first disease cell known from analysis to present EGFR at a density greater than normal, such as the U87wtEGFR or related lines that incorporate an altered EGFR such as U87EGFRvIII or the line A431, and a second, normal cell known from analysis to present EGFR at a normal density, such as primary human epidermal keratinocytes (∼ 20,000 EGFR/cell). The choice of epidermal keratinocytes as the reference, normal cell is prudent, given that marketed EGFR antibodies, such as cetuximab, are known to elicit severe skin rash side effects through their interaction with these cells. Any other human cell line that presents EGFR at normal density can be used, in the alternative.

The cell-based assay can use flow cytometry with appropriate EGFR antibody and labeled secondary antibody to report and measure binding affinity and avidity, as exemplified herein. In the alternative, selection of the desired antibody can be performed based on absolute binding affinities obtained for instance using surface plasmon resonance, also as exemplified herein.

For purposes of identifying disease cells that can be targeted by the present EGFR antibodies and bivalent fragments, the commercial test EGFR pharmDx™ (DAKO) can conveniently be used. This is a semi-quantitative immunohistochemical assay for determination of EGFR protein overexpression in colorectal tissues. Positive or negative results aid in the classification of abnormal cells/tissues and provide a basis for treatment with EGFR antibody.

The antibodies and binding fragments thus are useful both for diagnostic purposes, including sample testing and in vivo imaging, and for therapeutic purposes to treat diseases in which EGFR density is increased on disease cells.

For either purpose, the antibody or binding fragment can be conjugated to an appropriate agent, to form an immunoconjugate. Agents appropriate for treating disease include cytotoxic agents include chemotherapeutics and radiotherapeutics. For diagnostic purposes, appropriate agents are detectable labels that include radioisotopes, for whole body imaging, and radioisotopes, enzymes, fluorescent labels and the like for sample testing.

For therapy, the cytotoxin may be conjugated with the antibody or bivalent binding fragment through non-covalent interaction, but more desirably, are coupled by covalent linkage either directly or, more preferably, through a suitable linker. In a preferred embodiment, the conjugate comprises a cytotoxin and an antibody. Immunoconjugates of the antibody and cytotoxin are made using a variety of bifunctional protein coupling agents such as N-succinimidyl-3-(2-pyridyldithiol) propionate, iminothiolane, bifunctional derivatives of imidoesters such as dimethyl adipimidate HCL, active esters such as disuccinimidyl suberate, aldehydes such as glutaraldehyde, bis-azido compounds such as bis-(p-diazoniumbenzoyl)-ethylenediamine), diisocyanates such as toluene 2,6-diisocyanate, and bis-active fluorine compounds (such as 1,5-difluoro-2,4-dinitrobenzene). Carbon-14-labeled 1-isothiocyanobenzyl-3-methyldiethylene triaminepentaacetic acid (MX-DTPA) is a chelating agent suitable for conjugation of radio nucleotide to the antibody.

The cytotoxin component of the immunoconjugate can be a chemotherapeutic agent, a toxin such as an enzymatically active toxin of bacterial, fungal, plant or animal origin, or fragments thereof, or a small molecule toxin, or a radioactive isotope such as ²¹²Bi, ¹³¹I, ¹³¹In, ¹¹¹In, ⁹⁰Y, and ¹⁸⁶Re, or any other agent that acts to inhibit the growth or proliferation of a cancer cell.

Chemotherapeutic agents useful in the generation of such immunoconjugates include adriamycin, doxorubicin, epirubicin, 5-fluoroouracil, cytosine arabinoside ("Ara-C"), cyclophosphamide, thiotepa, busulfan, cytoxin, taxoids, e.g. paclitaxel, and docetaxel, taxotere, methotrexate, cisplatin, melphalan, vinblastine, bleomycin, etoposide, ifosgamide, mitomycin C, mitoxantrone, vincristine, vinorelbine, carboplatin, teniposide, daunomycin, carminomycin, aminopterin, dactinomycin, mitomycins, esperamicins, 5-FU, 6-thioguanine, 6-mercaptopurine, actinomycin D, VP-16, chlorambucil, melphalan, and other related nitrogen mustards. Also included are hormonal agents that act to regulate or inhibit hormone action on tumors such as tamoxifen and onapristone. Toxins and fragments thereof which can be used include diphtheria A chain, nonbonding active fragments of diphtheria toxin, cholera toxin, botulinus toxin, exotoxin A chain (from *Pseudomonas aeruginosa*), ricin A chain, abrin A chain, modeccin A chain, alpha-sarcin, Aleurites fordii proteins, dianthin proteins, *Phytolacca americana* proteins (PAPI, PAPII, and PAP-S), momordica charantia inhibitor, curcin, crotin, *Sapaonaria, officinalis* inhibitor, gelonin, saporin, mitogellin, restrictocin, phenomycin, enomycin, and the tricothcenes. Small molecule toxins include, for example, calicheamicins, maytansinoids, palytoxin and CC1065.

Therapeutic formulations of the antibody, bivalent fragment or the conjugate are prepared for storage by mixing the antibody or conjugate having the desired degree of purity with optional pharmaceutically acceptable carriers, excipients or stabilizers (Remington's Pharmaceutical Sciences, 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as serum, albumin, gelatin, or immunoglobulins; hydrophilic polymers such as polyvinylpyrrolidone; amino acids such as glycine, glutamine, asparagines, histidine, arginine or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN, PLURONICS or polyethylene glycol (PEG).

The active ingredients to be used for *in vivo* administration must be sterile. This is readily accomplished by filtration through sterile filtration membranes.

Sustained-release preparations may be prepared. Suitable examples of sustained-release include semipermeable matrices of solid hydrophobic polymers containing the antibody, which matrices are in the form of shapes articles, e.g., films or microcapsules. Examples of sustained-release matrices include polyesters, hydrogels (for example, poly (2-hydroxyethyl-methacrylate), polylactides (U.S. Pat. No. 3,773,919), copolymers of L-glutamic acid and ethyl-L-glutamate, non-degradable ethylene-vinyl acetate, degradable lactic acid-glycolic acid copolymers such as injectable microspheres composed of lactic acid-glycolic acid copolymer and leuprolide acetate, and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°-C-, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Administration "in combination with" one or more further therapeutic agents includes simultaneous (concurrent) and consecutive administration in any order. Other therapeutic regimens may be combined with the administration of the anti-cancer agents, e.g., antibodies or conjugates, of the instant invention. For example, the patient to be treated with such anti-cancer agents may also receive radiation therapy, such as external beam radiation. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M. C. Perry, Williams & Wilkins, Baltimore, Md. (1992). The chemotherapeutic agent may precede, or follow administration or the anti-tumor agent, e.g., antibody, or may be given simultaneously therewith. The antibody may be combined with any of the toxins described above with reference to the conjugates, or any other suitable drug particularly include irinotecan (CPT-11), cisplatin, cyclophosphamide, melphalan, dacarbazine, doxorubicin, daunorubicin, and topotecan, as well as tyrosine kinase inhibitors.

It may be desirable to also administer antibodies or conjugates against other tumor associated antigens or their ligands, such as antibodies which bind to the ErbB2, ErbB3, ErbB4, or vascular endothelial factor (VEGF), and/or antibodies that bind to EGF or TGFα. Alternatively, or in addition, two or more antibodies binding that same or two or more different antigens disclosed herein may be co-administered to the patient. Sometimes it may be beneficial to also administer one or more cytokines to the patient. In a preferred embodiment, the antibodies herein are co-administered with a growth inhibitory agent. For example, the growth inhibitory agent may be administered first, followed by an antibody of the present invention. However, simultaneous administration or administration of the antibody of the present invention first is also contemplated. Suitable dosages for the growth inhibitory agent are those presently used and may be lowered due to combined action (synergy) of the growth inhibitory agent and the antibody herein.

In another embodiment of the invention, an article of manufacture containing materials useful for the diagnosis or treatment of the disorders described herein is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or vial having a stopper pierceable by a hypodermic injection needle). The label on, or associated with, the container indicates that the composition is used for treating a cancer condition. The article of manufacture may further compromise a second container compromising a pharmaceutically-acceptable buffer, such as phosphate-buffered saline, Ringer's solution and dextrose solution. It may further include other matters desirable from a commercial and use standpoint, including other buffers, diluents, filters, needles, syringes, and package inserts with instructions for use.

An anti-cancer therapeutic according to the invention may be administered with a pharmaceutically-acceptable diluent, carrier, or excipient, in unit dosage form. -Any appropriate route of administration can be employed, for example, parenteral, intravenous, subcutaneous, intramuscular, intracranial, intraorbital, ophthalmic, intraventricular, intracapsular, intraspinal, intracisternal, intraperitoneal, intranasal, aerosol, or oral administration.

For the treatment of subjects presenting with cancer cells presenting EGFR at greater density than normal cells, the appropriate dosage of an anti-tumor agent, e.g., an antibody, fragment or conjugate, will depend on the type of disease to be treated, as defined above, the severity and course of the disease, whether the agent is administered for preventative or therapeutic purposes, previous therapy, the patients clinical history and response to the agent, and the discretion of the attending physician. The agent is suitably administered to the patient at one time or over a series of treatments. For example, depending on the type and severity of the disease, about 1 µg/kg to 15 mg/kg (e.g., 0.1-20 mg/kg) of antibody or conjugate is a candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

It will thus be appreciated that an effective amount of the antibody, fragment or immunoconjugate is an amount effective alone or as part of a treatment regimen that retards or inhibits the growth or proliferation of disease cells presenting with higher than normal EGFR density.

In embodiments, the present antibodies are administered by intravenous infusion, such as at an initial dose of 4 mg/kg over 90 minutes, then 2 mg/kg over 30 minutes, once weekly for 52 weeks, with follow up as required.

The antibody and bivalent fragments are useful in the treatment of a variety of cancers, to inhibit the growth or proliferation of cancer cells and tumours comprising them, including hematopoietic cell cancers and solid tumours. Conditions or disorders to be treated include benign or malignant tumors (e.g., renal, liver, kidney, bladder, breast, gastric, ovarian, colorectal, prostate, pancreatic, lung, vulva, and thyroid); hepatic carcinomas; sarcomas; glioblastomas; and various head and neck tumors; leukemias and lymphoid malignancies. In particular embodiments, the antibody or bivalent fragment are used in the treatment of such cancer cells that express high density EGFR, as determined by the screening assays herein described. In particular embodiments, the cancer cells are EGFR-presenting cancer cells that include head and neck cancers and especially squamous cell carcinoma of the head and neck, colorectal cancers, gastrointestinal cancers, brain tumours including glioblastomas, and tumours of the lung including non-small-cell lung carcinoma, and of the breast, pancreas, esophagus, kidney, ovary, cervix and prostate.

It will be appreciated that subjects who could benefit from the present method include mammals including humans as well as livestock, and pets.

Antibodies and bivalent fragments thereof that bind selectively to the target antigen, e.g. EGFR, are used, in accordance with an aspect of the invention, to screen cancer cells to detect those which present the EGFR antigen at high density. In a preferred embodiment, screening is applied to a sample of cancer cells taken from a subject that is a candidate for EGFR antibody therapy. Subjects testing positive for cancer cells that present the EGFR antigen at high density can then be scheduled for therapy with the present antibody or fragment, or an immunoconjugate thereof. Standard techniques, combined with the antibodies or other binding agents herein described, can be used to screen cancer cells. Desirably, the antibodies incorporate a detectable label. The label may be detectable by itself, (e.g., radio-isotope labels or fluorescent labels) or, in the case of an enzymatic label, may catalyze chemical alteration of a substrate compound or composition which is detectable. Radionuclides that can serve as detectable labels include, for example, 1-131, I-123,I-125, Y-90, Re-188, Re-186, At-211, Cu-67, Bi-212, and Pd-109.

*In situ* detection of the binding to cancer cells bearing high density EGFR can be performed, using the present antibody or fragment, by immunofluorescence or immunoelectron microscopy. For this purpose, a histological specimen is removed from the patient, and a labeled form of the present antibody is applied to it, preferably by overlaying the antibody on a biological sample. This procedure also allows for distribution of the EGFR antigen to be examined within biopsied tumour tissue, to reveal only those sites at which the antigen is presented at a density higher than normal. It will be apparent for those skilled in the art that a wide variety of histological methods are readily available for in situ detection.

More particularly, EGFR antibodies or binding fragments of the present invention may be used to monitor the presence or absence of antibody reactivity in a biological sample (e.g., a tissue biopsy, a cell, or fluid) using standard detection assays. Immunological assays may involve direct detection, and are particularly suited for screening large amounts of samples for the presence of cancer cells that overexpress EGFR. For example, antibodies may be used in any standard immunoassay format (e.g., ELISA, Western blot, immunoprecipitation, flow cytometry or RIA assay) to measure complex formation. Any appropriate label which may be directly or indirectly visualized may be utilized in these detection assays including, without limitation, any radioactive, fluorescent, chromogenic (e.g., alkaline phosphatase or horseradish peroxidase), or chemiluminescent label, or hapten (for example, digoxigenin or biotin) which may be visualized using a labeled, hapten-specific antibody or other binding partner (e.g., avidin). Exemplary immunoassays are described, e.g., in Ausubel et al., supra, Harlow and Lane, Antibodies: A Laboratory Approach, Cold Spring Harbor Laboratory, New York (1988), and Moynagh and Schimmel, Nature 400:105, 1999. For example, using the antibodies described herein, high density EGFR is readily detected at the cell surface using standard flow cytometry methods. Samples found to contain labeled complex compared to appropriate control samples are taken as indicating the presence of high density EGFR, and are thus indicative of a cancer or other disease amenable to treatment with the present antibodies.

The present antibody is produced suitably by recombinant DNA means, as exemplified herein. For production, there is provided a DNA molecule that encodes the heavy chain of the present antibody, and a DNA molecule that encodes the light chain thereof. The DNA further encodes any suitable signal peptide suitable for expression of a secretable chain precursor that enables proper externalization with folding and disulfide formation to elaborate the desired antibody as a secreted, dimerized and processed protein. To this end, the present invention provides, in one embodiment, a polynucleotide comprising a sequence that encodes the variable region of the light chain of a presently preferred EGFR antibody, as set out in SEQ ID No. 9 appearing at the end of the disclosure. Also provided, in another embodiment, is a polynucleotide comprising a sequence that encodes the variable region of the heavy chain of a presently preferred EGFR antibody, as set out in SEQ ID No. 10 also appearing at the end of the disclosure.

In more specific embodiments, the present invention provides a polynucleotide that encodes the entire light chain (SEQ ID No. 11) and the entire heavy chain (SEQ ID No. 14) of a preferred EGFR antibody of the present invention. These sequences also are provided at the end of this disclosure.

It will be appreciated that polynucleotide equivalents also can be used, in which synonymous codons are replaced within the sequences provided, to produce the present antibodies.

In embodiments, there are also provided vectors that comprise polynucleotides that encode the heavy chain or the variable region thereof and that encode the light chain or the variable region thereof. To express the antibodies, the polynucleotides are incorporated operably within expression vectors, i-.e., operatively linked to transcriptional and translational control sequences. Expression vectors include plasmids, retroviruses, cosmids, and the like. The expression vector and expression control sequences are chosen to be compatible with the expression host cell used. The antibody light chain gene and the antibody heavy gene can be inserted into separate vectors. In a preferred embodiment, both genes are inserted into the same expression vector. The antibody genes are inserted into the expression vector by standard methods (e.g., ligation of complementary restriction sites on the antibody gene fragment and vector, or blunt end ligation if no restriction sites are present).

A convenient vector is one that encodes a functionally complete human CH or CL immunoglobulin sequence, with appropriate restriction sites engineered so that any VH or VL sequence can be easily inserted and expressed, as described above. In such vectors, splicing usually occurs between the splice donor site in the inserted J region, and the splice acceptor site preceding the human C region, and also at the splice regions that occur within the human CH exons. Polyadenylation and transcription termination occur at native chromosomal sites downstream of the coding regions. The recombinant expression vector can also encode a signal peptide that facilitates secretion of the antibody chain from a host cell. The antibody chain gene may be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

Polynucleotides encoding the heavy chain and/or the light chain, and vectors comprising these can be used for transformation of a suitable mammalian host cell. Methods for introduction of heterologous polynucleotides into mammalian calls include dextran-mediated transfection, calcium phosphate precipitation, polybrene-mediated transfection, protoplast fusion, electroporation, encapsulation of the polynucleotide(s) in liposomes, biolistic injection and direct microinjection of the DNA into nuclei. In addition, polynucleotides may be introduced into mammalian cells by viral vectors.

Mammalian cell lines useful as hosts for expression of the antibody-encoding polynucleotides include many immortalized cell lines available from the American Type Culture Collection (ATCC). These include, inter alia, Chinese hamster ovary (CHO) cells, NSO, SP2 cells, HeLa cells, baby hamster kidney (BHK) cells, monkey kidney cells (COS, human hepatocellular carcinoma cells (e.g., Hep G2), A549 cells, 3T3 cells, and a number of other cell lines. Mammalian host cells include human, mouse, rat, dog, monkey, pig, goat, bovine, horse, and hamster cells. Cell lines of particular preference are selected through determining which cell lines have high expression levels. Other cell lines that may be used are insect cell lines, such as S19 cells, amphibian cells, bacterial cells, plant cells and fungal cells. When recombinant expression vectors encoding the heavy chain or antigen-binding portion thereof are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

It is likely that antibodies expressed by different cell lines or in transgenic animals will have different glycosylation from each other. However, all antibodies encoded by the polynucleotides provided herein, or comprising the amino acid sequences provided herein are part of the disclosure. Embodiments are now described in the following examples.

### Examples

The structure of cetuximab bound to EGFR [1] was used as starting point for mutant design. Mutations were introduced only in the CDR regions of the light and heavy chain. First, single-point mutations were generated and evaluated computationally. Virtual mutagenesis was carried out with optional conformational relaxation upon mutation by means of conformational sampling algorithms, such as Monte-Carlo minimization [2]. Prediction of antigen-antibody relative binding affinities between parent and mutant antibodies was carried out with binding affinity scoring functions, such as the solvated interaction energy (SIE) function [3]. Prediction of relative antigen-antibody association rates (kₒₙ) between parent and mutant antibodies was carried out with methods that evaluate long-range electrostatic interactions, such as HyPARE [4]. Candidate single-point mutants were the assembled into multiple-point mutants and re-scored for relative binding affinity.

Multiple-point mutants were generated by combining single-point mutants between light and heavy chains to achieve the targeted change in affinity. A requirement was to use as few single-point mutants as possible and to maximize the number of generated assembled antibodies. Another desirable feature was to generate a pool of mutants with reduced affinities due to either increased dissociation rates (k-_{off}) or to decreased association rates (k-ₒₙ). Among suitable candidate single-point mutations, those targeting distinct locations within the antibody-antigen interface, preferably at its periphery, were given higher priority.

### Preparation of Plasmids

All the cDNAs encoding the heavy and light chains of the antibodies were ordered from GeneArt (Regensburg Germany). The cDNAs were removed from the plasmid provided by GeneArt by digestion with *Hind*III and cloned into the *Hind*III site of plasmid pKCR5 previously dephosphorylated with calf intestinal phosphatase (NEB) to prevent recircularization. In pKCR5, transcription of the cDNA is under the control of the strong CR5 promoter, part of the cumate gene switch. The plasmid pKCR5 is available from the Biotechnology Research Institute, Montreal, Canada and is described by Mullick et al [6]. This 3.9 kb plasmid incorporates a HindIII in proper context with the CR5 promoter and a rabbit b-globin polyA, together with a B-lactamase gene for selection, and colEland f1 origins of replication. For transfection of CHO cells, all plasmids were isolated from large culture of *E. coli* using the Plasmid Maxi kit (Qiagen Inc, Mississauga, ON) according to the manufacturer's recommendation. Briefly, 200 ml of LB medium containing 100 µg/ml ampicillin were inoculated with a single fresh colony of E. coli and incubated overnight at 37°C with vigorous shaking (250 rpm). The bacteria were pelleted by centrifugation at 6000 x g, for 15 min, at 4°C and the plasmid was isolated using the protocols, buffers and columns provided by the kit. The pure plasmids was resuspended in sterile 50 mM TRIS, pH 8 and quantified by measuring the optical density at 260 nm.

### Cell line (CHO-cTA; clone 5F1) and growth conditions

The CHO-cTA cell line (Gaillet et al [5]; Mullick et al. [6]) used for transient transfection is a Chinese Hamster Ovary cell line (CHO) adapted to grow in suspension and in protein-free medium. The cell line stably expresses the cumate transactivator (cTA) which activates transcription by binding to the CR5 promoter. The CHO-cTA are maintained in CD-CHO medium (Invitrogen, CDCHO 10743), supplemented with 4 mM glutamine, 50 µg/mL and dextran sulfate (Amersham Pharmacia Biotech) at 37°C under an atmosphere of 5% CO₂. When the cells reach a concentration of 1.0 X 10⁶ cells/ml (on average three times a week) they are passaged by diluting them to a concentration of 5.0 x 10⁴ cells/ml using fresh medium.

### Transient transfection of CHO-cTA

Before transfection, the cells were washed with PBS and resuspended at a concentration of 2.5 X 10⁶ cell/ml in growth medium without dextran sulfate for 3 h in suspension culture. 50 ml of cells were transfected by adding slowly 2.5 ml of a CDCHO medium supplemented with 1 µg/ml of plasmid and 5 µg/ml. polyethylenimine (PEI Max; Polysciences). After 2 h, the cells were transferred at 30°C. The next days, 50 µg/ ml of dextran sulfate was added to the cells and they were incubated at 30°C for a total of 4 days. The supernatant was clarified by centrifugation and filtered through a 0.22 µM filter and transferred at -80°C until further analysis.

### Polyacrylamide gel electrophoresis (SDS-PAGE)

Known amounts of supernatant were resuspended into an equal volume of Laemmli 2X and heated at 95°C for 5 min and chilled on ice. The samples were then separated on a polyacrylamide Novex 10% Tris-Glycine gel (Invitrogen Canada Inc., Burlington, ON). A standard curve was made by adding known amount of purified human IgG. The gel was then stained using a solution of Coomassie Fluor™-Orange (Molecular Probes, Eugene OR) according to the manufacturer's recommendations. The signal was visualized and quantified using the Typhoon Scanner.

### Western blot analysis

Known amounts of supernatant were separated on a SDS-PAGE as described above and then transferred onto a Hybond-N nitrocellulose membrane (Amersham Bioscience Corp., Baie d'Urfé, QC) for 1 h at 275 mA. The membrane was blocked for 1 h in 0.15% Tween 20, 5% skimmed milk in PBS and incubated for 1 h with an anti-human IgG conjugated to Cy5 (Jackson, Cat# 109-176-099). The signal was revealed by scanning with the Typhoon Trio+ (Amersham Biosciences, GE Healthcare).

### ELISA

96 wells/plates were coated with 50 µl of AffiniPure goat anti-human IgG, (Jackson Immuno Research) and incubated overnight at 4º°C. The wells were washed with PBS and incubated for 30 min at 37º°C with 100 µl of 1% BSA in PBS at 37ºC. 25 µl of samples diluted with 1% BSA in PBS were added to the wells, which were incubated for 2 h at 37º°C. The wells were washed with 0.05% Tween 20 in PBS and incubated with an alkaline Phosphatase-conjugated AffiniPure Goat Anti-Human IgG (H+L) (Jackson Immuno Research) for 1 h at 37°C. The wells were washed with 0.05% Tween 20 in PBS, followed by PBS. The signal was revealed by incubation with PNPP for 30 min at 37°C. The signal intensity was measure at 405 nm. A standard curve was made using known amount of purified antibody (IgG1, kappa from myeloma plasma (Athens Research Technology).

### Purification of antibody

The supernatant was concentrated with an Amicon Ultra (Ultracel-50K) at 1500 rpm to a volume of 500 µl. The wild type and mutants, antibodies were purified using the ProPur protein A mini spin columns (Nunc) according to the manufacture's recommendations. The purified antibodies were then desalted and resuspended in PBS using the desalting column PD-10 (GE Healthcare). The antibodies were then concentrated by centrifugation on an Amica Ultra 100,000 MWCO membrane. The purified antibodies were quantified by reading the optical density at 280 nm using the Nanodrop spectrophotometer. The purified antibodies were kept frozen at -20°C in 50% glycerol.

### In vitro binding by Surface Plasmon Resonance

Kinetic and affinity analysis was carried out using a BioRad Proteon surface plasmon resonance instrument. The running buffer for all steps was 10 mM HEPES, 150 mM NaCl, 3.5 mM EDTA and 0.05% Tween20 at pH 7.4. An antibody capture sensorchip was prepared by injecting 6.5 µg/mL of anti-human Fc (Jackson Immunochemicals Inc.) in 10 mM sodium acetate pH 4.5 at flow rate 25 µL/min over a GLM sensorchip (BioRad Inc.) that had previously been activated with a 1/10 dilution of sNHS/EDC (BioRad Inc.) until the surface was saturated (approximately 5000 RUs). This procedure was carried out in the analyte direction to ensure all of the inter spots for referencing have immobilized anti-mouse Fc. Wild-type cetuximab and variants were captured in the ligand direction by injecting 100 µL of 4% culture supernatants or purified samples in running buffer at flow rate of 25 µL/min until 400 to 800 resonance units have been captured. This was immediately followed by two pulses of running buffer in the analyte direction, 50 µL each at flow rate 100 µL/min to stabilize the baseline. Next, the simultaneous injection of 100 µL of five EGFR ectodomain (EGFRed) concentrations (3-fold dilutions of 20 nM to 1000 nM EGFR depending on the affinity of the cetuximab variant) and buffer blank at a flow rate of 50 µL/min with a 600 s dissociation was carried out to analyze the EGFRed-antibody interaction. Kinetic rate constants (on- and off-rates) and affinity constants were generated from the aligned and double referenced sensorgrams with the Langmuir binding model using BioRad Proteon Manager software v3.1. Mutants with fast on- and off-rates had their affinity constants determined using the equilibrium fit model which uses plateau values from the sensorgrams to generate a binding isotherms for Kd constant determination.

### Cell culture

The U87MG glioblastoma cell line was obtained from ATCC (HTB-14). A stably transfected full length wt EGFR or a deleted version of EGFR vIII-overexpressing cell line variants were gifts from W. Cavanee, Ludwig Institute for Cancer Research, University of California, San Diego). The human epidermoid A431 cell line was obtained from ATCC (CRL-1555). Cell lines were maintained in DMEM (Gibco) containing 10% fetal bovine serum (Gibco). Primary adult human epithelial keratinocytes were obtained from ScienCell (Catalog # 2110) and cultured using manufacturer's recommended Keratinocyte Medium (KM, Cat. No. 2101). Generally cells were passaged once or twice a week and used within 4-6 weeks for all experiments.

### Detection of antibody binding to surface EGFR level by flow cytometry

Prior to analysis, cells were plated such that they were not more than 80% confluent on the day of analysis. Tumor (U87 MG derivatives, A431) or normal (human epidermal keratinocytes) cell were washed in PBS and harvested by the addition of cell dissociation buffer (Sigma.). A cell suspension containing 2.5 x 10⁵ cells (in 500 µl corresponding cell culture media) was incubated with various concentrations (0.01-100 ug/ml) of anti - EGFR antibodies for 2 h at 4º°C (to prevent internalization). Following 1 wash with cell culture media, primary antibody was incubated with 2 µg Dylight 488 conjugated AffiniPure goat anti-human IgG Alexa 488 secondary antibody (Jackson Immuno Research 109-487-003) in 100 µl media for 1h at 4º°C. Cells were then pelleted and stored on ice until ready to be analyzed by flow cytometry. Prior to analysis, cell pellets were resuspended in 300-500 µl media and filtered through a 50 µm nylon mesh filter to remove cell aggregates. Flow cytometry analyses were performed on 10,000 viable cells gated on forward scattering, side scattering parameters and propidium iodide dye exclusion using a BD LSRII flow Cytometer (Becton-Dickinson Biosciences, CA, USA) and a standard filter set using BD FACSDiva™ acquisition software, according to manufacturer's instructions.

Specific antibody binding was calculated as the mean fluorescent intensity of binding to each antibody after background level subtraction of the mean fluorescent intensity of binding in the absence of primary antibody (but containing secondary detection antibody). An alternative approach was used to calculate specific antibody binding on cells, i.e. it was calculated as fold-binding over background by dividing the mean fluorescent intensity in the presence of primary antibody by the mean fluorescent intensity obtained in the absence of primary antibody (but containing secondary antibody). To examine the binding selectivity of the antibodies, the value of antibody binding to tumor (overexpressing EGFR) was divided by the binding observed with cells not overexpressing EGFR. This parameter, named the ratio of binding, was calculated and compared to that seen with wild type antibody. A commercial source of cetuximab (Merck KGaA) was used as a benchmark for comparison purposes.

### Evaluation of antibody-mediated cytotoxicity as antibody-drug conjugates

In this set of experiments, primary antibodies (typically 1 nM in concentration) were incubated with 2 nM anti-human secondary antibody that was chemically conjugated with saporin toxin (from Advanced Targeting Systems, San Diego, CA), a ribosome inactivating enzyme that needs to be internalized to cause cell death. The antibody complex was then added to the cell types indicated (plated in triplicate) and their effects on cell viability measured after 72 h incubation at 37°C. EGFR directed cytotoxicity can be quantitated following evaluation with controls for non-specific cytotoxicity (no primary antibody or an irrelevant primary antibody (control human IgG) were used to assess non-specific cytotoxicity). Cell viability can be measured using standard techniques, including the use of sulforhodamine B.

### Results:

### 1. Production and Purification of EGFR Antibodies

Nine cDNAs corresponding to the coding sequence of the EGFR antibodies were synthesized (GeneArt). All the cDNAs were cloned into the HindIII site of pKCR5, an expression vector regulated by the cumate-switch (pKCR5 vector For each antibody, 50 ml of CHOcTA (expressing the cumate transactivator, cTA) were transfected with various combinations of heavy and light chain. Four days after transfection the supernatant was analyzed by SDS-PAGE, Western Blot and ELISA.

Table 3 below summarizes quantification of the antibodies produced by transient transfection in CHOcTA cells, done by ELISA and by western blot using a purified human IgG1 as standard.

**Table 3**

| Mutants | | Quantification by Western blot ( mg/L ) | Quantification by E.L.I.S.A ( mg/L ) |
|---|---|---|---|
| **HC_LC** | wt_HC + wt_LC | 13.72 | 10.09 |
| **HC_ 1** | wt_HC + LC_E53K | 6.02 | 3.44 |
| **HC_ 2** | wt_HC+LC_W94A | 5.34 | 5.97 |
| **3_ LC** | HC_Y101A + wt_LC | 10.73 | 6.57 |
| **3_1** | HC_Y101A + LC_E53K | 1.72 | 3.62 |
| **3_2** | HC_Y101A + LC_W94A | 6.24 | 5.13 |
| **4_ LC** | HC_Y102A + wt_LC | 6.77 | 6.64 |
| **4_1** | HC_Y102A + LC_E53K | 6.59 | 6.19 |
| **4_2** | HC_Y102A + LC_W94A | 9.17 | 7.16 |
| **5_LC** | HC_D103N + wt_LC | 18.46 | 9.58 |
| **5_1** | HC_D103N + LC_E53K | 2.52 | 6.59 |
| **5_2** | HC_D103N + LC_W94A | 21.05 | 13.59 |
| **6_ LC** | HC_D58N_D103N + wt_LC | 18.55 | 15.71 |
| **6_1** | HC_D58N_D103N + LC_E53K | 6.47 | 7.03 |
| **6_2** | HC_D58N_D 103N + LC_W94A | 29.13 | 20.03 |
| **7_ LC** | HC_D58N_D103N_E105Q + wt_LC | 16.36 | 11.04 |
| **7**_**1** | HC_D58N_D103N_E105Q + LC_E53K | 9.86 | 5.82 |
| **7**_**2** | HC_D58N_D103N_E105Q + LC_W94A | 17.38 | 12.41 |

The 2 wild-type chains and 7 mutant chains were purified by chromatography using protein A. The purified proteins were quantified by OD₂₈₀ (NanoDrop). The purified antibodies were analyzed by non-denaturing and denaturing SDS-PAGE.

### 2. Binding affinity determination of EGFR antibodies by SPR

The SPR results are provided in Table 4 and Table 4-1 below:

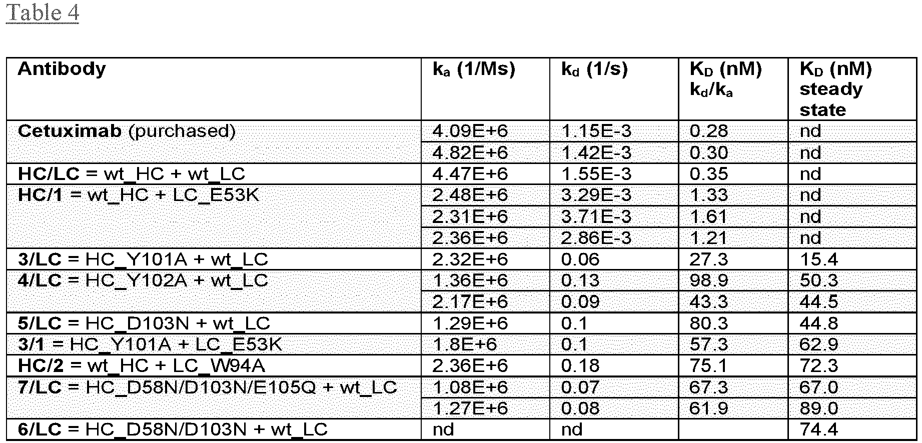

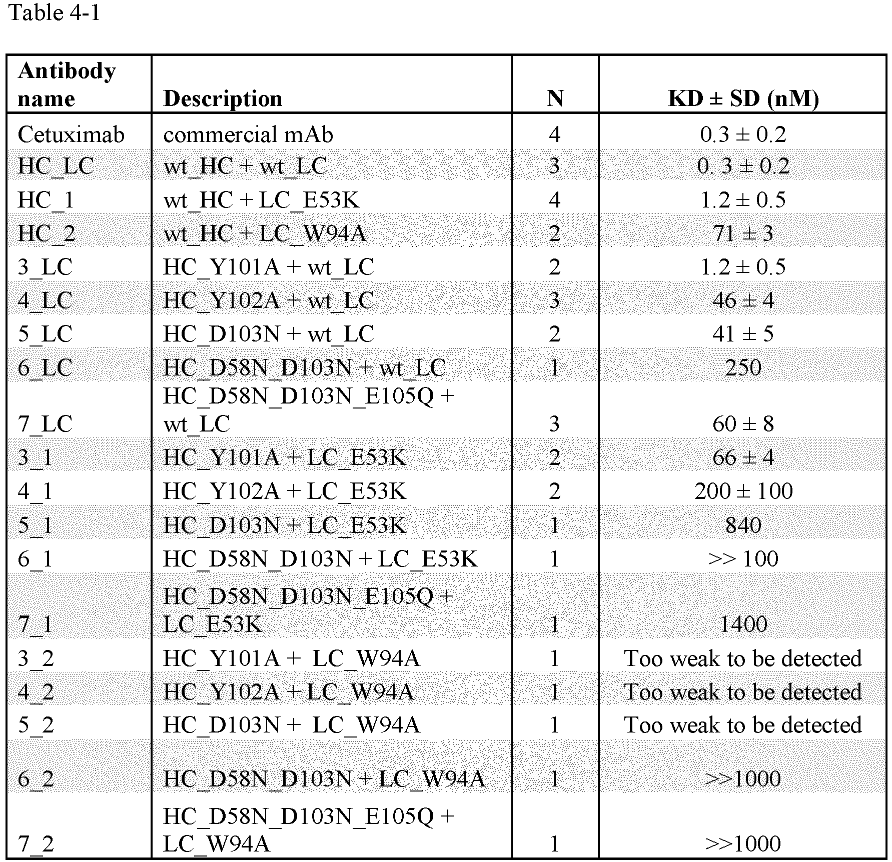

Table 4-1 provides SPR-based affinity determinations that have either been refined or are additional to those provided in Table 4.

The results indicated that approximately 50% of the cetuximab variants did not have any detectable activity at the 100 nM EGFR tested (data not shown). Of those that showed binding activity (Table 4), only the wild-type (HC/LC) and mutant HC-1 had a moderately-slow off rate. All of the other variants with activity (3-LC, 4-LC, 4-1, 5-LC, 6-C, 7-LC, 3-1 and HC-2) had both a fast association and dissociation from the flowing EGFRed. Affinity constants (Kd's) were determined from the ratio of the kinetic rates (kd s-1 / ka s-1M-1) using a 1:1 Langmuir binding model where amenable, otherwise affinity constants were determined from an equilibrium fit using plateau binding values only. Mutants 6-2 and 7-2 showed very weak binding at 1000 nM EGFR but were not quantifiable.

### 3. Binding of EGFR antibodies to various cell lines as determined by indirect flow cytometry

Figure 1 depicts graphs showing binding of antibodies to cell surface EGFR present on the surface of (A) parental U87MG cells, (B) U87 cells overexpressing wt EGFR, (C) U87 cells overexpressing EGFR vIII and (D) primary human epidermal keratinocytes (HEK), at 1 and 10 µg/ml mAb (A-C) or 0.1 and 1 µg/ml mAb (D). These were compared to wt mAb (HC-LC, set arbitrarily to 100%). In Figure 1-1 (A and B), the same plus additional results are presented differently, i.e. all binding is divided by background binding (that is, is expressed is as a fold change over background binding) rather than background binding being subtracted from all binding values (as in Figure 1). This data analysis approach de-emphasizes variations caused by small changes in background binding. As expected, these results demonstrate less binding of anti-EGFR mAbs to parental U87 cells or HEK cells compared to tumor cells which overexpress EGFR. Importantly, these results demonstrate a greater reduction in binding of some anti-EGFR mAb variants to cells expressing lower EGFR levels (parental U87 or HEK cells) as compared to U87 cells overexpressing EGFR.

### 4. Evaluation of antibody binding to tumor and normal cell lines

Figure 2 is a graph representing binding selectivity of antibodies. The ratio of antibody binding (with background subtracted) to EGFR overexpressing cells [U87MGwtEGFR or A431 cells (which naturally overexpress wt EGFR)] relative to antibody binding to normal HEK cells was calculated and compared to that seen with wild type antibody (ratio set arbitrarily to 1 for wt antibody). This result clearly shows that some of the EGFR mAbs exhibit a better ratio of binding to tumor relative to normal HEK cells (e.g. mutant HC-2 exhibits a 20-fold better ratio, and mutant 3-1 exhibits a 40-50-fold better ratio of binding to tumor versus normal cells). In Figure 2-1, the same results as in Figure 2 are shown using a different data presentation approach, i.e. all binding is divided by background. These results also clearly show that some of the EGFR mAbs exhibit a better ratio of binding to tumor cells that overexpress EGFR relative to normal HEK cells (e.g. mutant HC-2 exhibits 80-100 fold differential binding, and mutant 3-1 exhibits 120-140 fold differential binding to tumour cells versus normal cells, whereas wt antibody (HC-LC) exhibits 12-15 fold differential binding to tumour cells versus normal cells. In other words, HC-2 exhibits an ∼6-fold better ratio of binding, and mutant 3-1 exhibits an ~9-fold better ratio of binding to tumor than normal cells. The pattern of binding specificity was similar amongst the tumor cell lines analyzed (U87MGwt EGFR and A431) suggesting that the selectivity of binding is universally high for tumor cells overexpressing EGFR (~2 million receptors per cell or more).

It will further be appreciated from the results shown in Figure 3 that there is a greater reduction in binding of some anti-EGFR mAb variants to cells expressing (A) lower EGFR levels (parental U87) as compared to (B) U87 cells overexpressing EGFR.

Also, as shown in Figure 4, it is clear that the ratio of antibody binding to EGFR overexpressing cells (U87MGwtEGFR) relative to antibody binding to parental U87MG cells was improved in most cases by 2-4 fold. That is, a ratio of 11 for wild type antibody binding to U87MGwtEGFR cells versus parental cells, and ratios up to 35 for certain mutated antibodies, e.g. mutant 7-LC and 4-LC, were observed. Antibody 6-2 and 7-2 exhibited no detectable binding to EGFR on either cell type at the concentrations used (1 µg/ml).

Finally, in Figure 5 it is shown and confirmed that some mutant antibodies can bind to EGFR and deliver a protein toxin, in this case saporin. Mutant antibodies 6-2 and 7-2 exhibited cytotoxicity similar to that seen with the non-specific controls, which is not unexpected since they do not detectably bind EGFR on the surface of these cells (Figure 3). Notably, in comparison to the cytotoxicity profile seen for the wt EGFR MAb (HC/LC), antibodies 6-LC, 7-LC and 4-1 exhibited decreased cytotoxicity on cells with low levels of EGFR [human epidermal keratinocytes (HEK) and parental U87 cells] with little decrease in cytotoxicity on U87 cells overexpressing wild type EGFR.

In summary, this data indicates that mutant antibodies can be generated that bind highly selectively to cells that present EGFR at abnormally high density, and that these antibodies may be useful in oncology and other diseases as antibody-drug conjugates with broad therapeutic windows, and/or as diagnostic agents for the detection of EGFR overexpressing cells.

### REFERENCES

1. Li S, Schmitz KR, Jeffrey PD, Wiltzius JJ, Kussie P, Ferguson KM (2005) Cancer Cell 7:301-311
2. Li Z, Scheraga HA (1987) Proc. Natl. Acad. Sci. U.S.A. 84:6611-6615.
3. Naim M, Bhat S, Rankin KN, Dennis S, Chowdhury SF, Siddiqi I, Drabik P, Sulea T, Bayly CI, Jakalian A, Purisima EO (2007) J. Chem. Inf. Model. 47:122-133.
4. Selzer T, Albeck S, Schreiber G (2000) Nat. Struct. Biol. 7:537-541.
5. Gaillet, B., R.Gilbert, R.Amziani, C.Guilbault, C.Gadoury, A.W.Caron, A.Mullick, A. Garnier, and B.Massie. 2007. High-Level Recombinant Protein Production in CHO Cells Using an Adenoviral Vector and the Cumate Gene- Switch. Biotechnol. Prog. 23-200-209
6. Mullick, A., Y.Xu, R.Warren, M.Koutroumanis, C.Guilbault, S.Broussau, F.Malenfant, L.Bourget, L.Lamoureux, R.Lo, A.W.Caron, A.Pilotte, and B.Massie. 2006. The cumate gene-switch: a system for regulated expression in mammalian cells. BMC Biotechnol. 6.43

Polynucleotides encoding the various mutant antibody chains are provided below. Substituted codons are shaded, and HindIII sites are highlighted:
**Light chain wild-type (shown here with the signal peptide) [SEQ ID No. 11]:** 
GTTTAAACGAATTCGCCCTTGAGGTACCAAGCTTGCCACCATGGTGCTGCAGACCCAGGT
GTTCATCTCCCTGCTGCTGTGGATCTCTGGCGCCTACGGCGACATCCTGCTGACCCAGTC
CCCCGTGATCCTGTCCGTGTCCCCTGGCGAGCGGGTGTCCTTCTCTTGCCGGGCCTCCCA
GTCCATCGGCACCAACATCCACTGGTATCAGCAGCGGACCAACGGCTCCCCTCGGCTGCT
GATCAAGTACGCCTCCGAGTCTATCTCCGGCATCCCTTCCCGGTTCTCCGGCTCTGGCTC
CGGCACCGACTTCACCCTGTCCATCAACTCCGTGGAGTCCGAGGATATCGCCGACTACTA
CTGCCAGCAGAACAACAACTGGCCTACCACCTTCGGCGCTGGCACCAAGCTGGAACTGAA
GCGGACCGTGGCCGCTCCTTCCGTGTTCATCTTCCCACCTTCCGACGAGCAGCTGAAGTC
CGGCACCGCCTCTGTGGTGTGCCTGCTGAACAACTTCTACCCTCGGGAGGCCAAGGTGCA
GTGGAAGGTGGACAACGCCCTGCAGTCCGGCAACTCCCAGGAATCCGTCACCGAGCAGGA
CTCCAAGGACTCTACCTACTCCCTGTCCTCCACCCTGACCCTGTCCAAGGCCGACTACGA
GAAGCACAAGGTGTACGCCTGCGAAGTGACCCACCAGGGCCTGTCCAGCCCTGTGACCAA
GTCCTTCAACCGGGGCGAGTGCTGAAAGCTTGAGCTCAGTAAGGGCGAATTCGCGGCCGC
**Light chain E58K mutant (shown here with the signal peptide) [SEQ ID No. 12]:** 
CGGAAGGCCCATGAGGCCAGTTAATTAAGAGGTACCAAGCTTGCCACCATGGTGCTGCAG
ACCCAGGTGTTCATCTCCCTGCTGCTGTGGATCTCTGGCGCCTACGGCGACATCCTGCTG
ACCCAGTCCCCCGTGATCCTGTCCGTGTCCCCTGGCGAGCGGGTGTCCTTCTCTTGCCGG
GCCTCCCAGTCCATCGGCACCAACATCCACTGGTATCAGCAGCGGACCAACGGCTCCCCT
CGGCTGCTGATCAAGTACGCCTCCAAGTCTATCTCCGGCATCCCTTCCCGGTTCTCCGGC
TCTGGCTCCGGCACCGACTTCACCCTGTCCATCAACTCCGTGGAGTCCGAGGATATCGCC
GACTACTACTGCCAGCAGAACAACAACTGGCCTACCACCTTCGGCGCTGGCACCAAGCTG
GAACTGAAGCGGACCGTGGCCGCTCCTTCCGTGTTCATCTTCCCACCTTCCGACGAGCAG
CTGAAGTCCGGCACCGCCTCTGTGGTGTGCCTGCTGAACAACTTCTACCCTCGGGAGGCC
AAGGTGCAGTGGAAGGTGGACAACGCCCTGCAGTCCGGCAACTCCCAGGAATCCGTCACC
GAGCAGGACTCCAAGGACTCTACCTACTCCCTGTCCTCCACCCTGACCCTGTCCAAGGCC
GACTACGAGAAGCACAAGGTGTACGCCTGCGAAGTGACCCACCAGGGCCTGTCCAGCCCT
GTGACCAAGTCCTTCAACCGGGGCGAGTGCTGAAAGCTTGAGCTCATGGCGCGCCTAGGC
CTTGACGGCCTTCCG
**Light chain W94A mutant (shown here with the signal peptide): [SEQ ID No.13]:** 
CGGAAGGCCCATGAGGCCAGTTAATTAAGAGGTACCAAGCTTGCCACCATGGTGCTGCAG
ACCCAGGTGTTCATCTCCCTGCTGCTGTGGATCTCTGGCGCCTACGGCGACATCCTGCTG
ACCCAGTCCCCCGTGATCCTGTCCGTGTCCCCTGGCGAGCGGGTGTCCTTCTCTTGCCGG
GCCTCCCAGTCCATCGGCACCAACATCCACTGGTATCAGCAGCGGACCAACGGCTCCCCT
CGGCTGCTGATCAAGTACGCCTCCGAGTCTATCTCCGGCATCCCTTCCCGGTTCTCCGGC
TCTGGCTCCGGCACCGACTTCACCCTGTCCATCAACTCCGTGGAGTCCGAGGATATCGCC
GACTACTACTGCCAGCAGAACAACAACGCCCCTACCACCTTCGGCGCTGGCACCAAGCTG
GAACTGAAGCGGACCGTGGCCGCTCCTTCCGTGTTCATCTTCCCACCTTCCGACGAGCAG
CTGAAGTCCGGCACCGCCTCTGTGGTGTGCCTGCTGAACAACTTCTACCCTCGGGAGGCC
AAGGTGCAGTGGAAGGTGGACAACGCCCTGCAGTCCGGCAACTCCCAGGAATCCGTCACC
GAGCAGGACTCCAAGGACTCTACCTACTCCCTGTCCTCCACCCTGACCCTGTCCAAGGCC
GACTACGAGAAGCACAAGGTGTACGCCTGCGAAGTGACCCACCAGGGCCTGTCCAGCCCT
 
GTGACCAAGTCCTTCAACCGGGGCGAGTGCTGAAAGCTTGAGCTCATGGCGCGCCTAGGC
CTTGACGGCCTTCCG
**Heavy chain wild-type (shown here with the signal peptide) [SEQ ID No. 14]:** 
CGAATTGAAGGAAGGCCGTCAAGGCCGCATGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCGACTACAACACCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACCTACTACGACTACGAG
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCCTGGGCCTCATGGGCCTTCCTTTCACTGCC
**Heavy chain Y101A mutant (shown here with the signal peptide) [SEQ ID No.15]:** 
CGGAAGGCCCATGAGGCCAGTTAATTAAGAGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCGACTACAACACCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACC[?][?][?]TACGACTACGAG
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
 
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCATGGCGCGCCTAGGCCTTGACGGCCTTCCG
**Heavy chain Y102A mutant (shown here with the signal peptide) SEQ ID No.16:** 
CGGAAGGCCCATGAGGCCAGTTAATTAAGAGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCGACTACAACACCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACCTAC[?][?][?]GACTACGAG
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCATGGCGCGCCTAGGCCTTGACGGCCTTCCG
**Heavy chain D103N mutant (shown here with the signal peptide) [SEQ ID No.17]:** 
CGGAAGGCCCATGAGGCCAGTTAATTAAGAGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCGACTACAACACCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACCTACTACAACTACGAG
 
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCATGGCGCGCCTAGGCCTTGACGGCCTTCCG
**Heavy chain D58N/D103N mutant (shown here with the signal peptide) [SEQ ILD No.18]:** 
CGGAAGGCCCATGAGGCCAGTTAATTAAGAGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCAAGTACAACACCCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACCTACTACAAGTACGAG
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCATGGCGCGCCTAGGCCTTGACGGCCTTCCG
**Heavy chain D58N/D103N/E105Q mutant (shown here with the signal peptide) [SEQ ILD No. 19]:**

CGGAAGGCCCATGAGGCCAGTTAATTAAGAGGTACCAAGCTTGCCACCATGGACTGGACC
TGGCGGATCCTGTTTCTGGTGGCCGCTGCTACCGGCACACACGCCCAGGTGCAGCTGAAG
CAGTCTGGCCCTGGCCTGGTGCAGCCTTCCCAGTCCCTGTCCATCACCTGTACCGTGTCC
GGCTTCTCCCTGACCAACTACGGCGTGCACTGGGTGCGCCAGTCTCCAGGCAAGGGCCTG
GAATGGCTGGGAGTGATTTGGTCCGGCGGCAACACCAAGTACAACACCCCTTTCACCTCC
CGGCTGTCCATCAACAAGGACAACTCCAAGTCCCAGGTGTTCTTCAAGATGAACTCCCTG
CAGTCCAACGACACCGCCATCTACTACTGCGCCAGGGCTCTGACCTACTACAACTACGAG
TTCGCCTACTGGGGCCAGGGCACCCTGGTGACCGTGTCCGCCGCTTCCACCAAGGGCCCT
AGCGTGTTCCCTCTGGCCCCTTCCAGCAAGTCTACCTCTGGCGGCACCGCTGCTCTGGGC
TGCCTGGTGAAGGACTACTTCCCTGAGCCTGTGACAGTGTCCTGGAACTCTGGCGCCCTG
ACCTCCGGAGTGCACACCTTCCCTGCTGTGCTGCAGTCCTCCGGCCTGTACTCCCTGTCC
TCCGTGGTGACAGTGCCTTCCTCCAGCCTGGGCACACAGACCTACATCTGCAACGTGAAC
CACAAGCCTTCCAACACCAAGGTGGACAAGCGGGTGGAGCCTAAGTCCTGCGACAAGACC
CACACCTGTCCTCCATGCCCTGCCCCTGAGCTGCTGGGCGGACCCTCCGTGTTCCTGTTC
CCTCCAAAGCCTAAGGACACCCTGATGATCTCCCGGACCCCTGAAGTGACCTGCGTGGTG
GTGGACGTGTCCCACGAGGATCCTGAAGTGAAGTTCAATTGGTACGTGGACGGCGTGGAG
GTGCACAACGCCAAGACCAAGCCTCGGGAGGAACAGTACAACTCCACCTACCGGGTGGTG
TCCGTGCTGACCGTGCTGCACCAGGACTGGCTGAACGGCAAAGAGTACAAGTGCAAGGTC
TCCAACAAGGCCCTGCCTGCCCCTATCGAAAAGACCATCTCCAAGGCCAAGGGCCAGCCT
CGGGAACCTCAGGTGTACACACTGCCTCCCAGCAGGGACGAGCTGACCAAGAACCAGGTG
TCCCTGACCTGTCTGGTGAAGGGCTTCTACCCTTCCGATATCGCCGTGGAGTGGGAGTCT
AACGGCCAGCCTGAGAACAACTACAAGACCACCCCTCCTGTGCTGGACTCCGACGGCTCC
TTCTTCCTGTACTCCAAACTGACCGTGGACAAGTCCCGGTGGCAGCAGGGCAACGTGTTC
TCCTGCTCCGTGATGCACGAGGCCCTGCACAACCACTACACCCAGAAGTCCCTGTCTCTG
TCCCCTGGCAAGTGAAAGCTTGAGCTCATGGCGCGCCTAGGCCTTGACGGCCTTCCG

Amino acid sequences constituting the antibody wild type and mutant chains are provided below. The signal peptide is indicated using lower case letters, and is not included in the residue numbering. Mutated positions are bolded in mutant sequences.
**Light chain wild-type [SEQ ID No. 20]:** 
mvlqtqvfisllllwisgaygDILLTQSPVILSVSPGERVSFSCRASQSIGTNIHWYQQRT
NGSPRLLIKYASESISGIPSRFSGSGSGTDFTLSINSVESEDIADYYCQQNNNWPTTFGA
GTKLELKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
**Light chain E58K mutant [SEQ ID No. 21]:** 
mvlqtqvfisllllwisgaygDILLTQSPVILSVSPGERVSFSCRASQSIGTNIHWYQQRT
NGSPRLLIKYASKSISGIPSRFSGSGSGTDFTLSINSVESEDIADYYCQQNNNWPTTFGA
GTKLELKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
**Light chain W94A mutant [SEQ ID No. 22]:** 
mvlqtqvfisllllwisgaygDILLTQSPVILSVSPGERVSFSCRASQSIGTNIHWYQQRTN
GSPRLLIKYASESISGIPSRFSGSGSGTDFTLSINSVESEDIADYYCQQNNNAPTTFGAGT
KLELKRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
**Heavy chain wild-type [SEQ ID No. 23]:** 
mdwtwrilflvaaatgthaQVQLKQSGPGLVQPSQSLSITCTVSGFSLTNYGVHWVRQSP
GKGLEWLGVIWSGGNTDYNTPFTSRLSINKDNSKSQVFFKMNSLQSNDTAIYYCARALTY
YDYEFAYWGQGTLVTVSAASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
**Heavy chain Y101A mutant [SEQ ID No. 24]:** 
mdwtwrilflvaaatgthaQVQLKQSGPGLVQPSQSLSITCTVSGFSLTNYGVHWVRQSPG
KGLEWLGVIWSGGNTDYNTPFTSRLSINKDNSKSQVFFKMNSLQSNDTAIYYCARALTAYD
YEFAYWGQGTLVTVSAASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCDKT
HTCPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK
**Heavy chain Y102A mutant [SEQ ID No. 25]:** 
mdwtwrilflvaaatgthaQVQLKQSGPGLVQPSQSLSITCTVSGFSLTNYGVHWVRQSPGK
GLEWLGVIWSGGNTDYNTPFTSRLSINKDNSKSQVFFKMNSLQSNDTAIYYCARALTYADYE
FAYWGQGTLVTVSAASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKRVEPKSCDKTHTCPPC
PAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRWQ
QGNVFSCSVMHEALHNHYTQKSLSLSPGK
**Heavy chain D103N mutant [SEQ ILD No. 26]:** 
mdwtwrilflvaaatgthaQVQLKQSGPGLVQPSQSLSITCTVSGFSLTNYGVHWVRQSPGKGL
EWLGVIWSGGNTDYNTPFTSRLSINKDNSKSQVFFKMNSLQSNDTAIYYCARALTYYNYEFAYW
GQGTLVTVSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSV
MHEALHNHYTQKSLSLSPGK
**Heavy chain D58N/D103N mutant [SEQ ILD No. 27:** 
mdwtwrilflvaaatgthaQVQLKQSGPGLVQPSQSLSITCTVSGFSLTNYGVHWVRQSPGKGLE
WLGVIWSGGNTNYNTPFTSRLSINKDNSKSQVFFKMNSLQSNDTAIYYCARALTYYNYEFAYWGQ
GTLVTVSAASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCSVMHEALHN
HYTQKSLSLSPGK
**Heavy chain D58N/D103N/E105Q mutant [SEQ ILD No. 28]:**

mdwtwrilflvaaatgthaQVQLKQSGPGLVQPSQSLSITCTVSGFSLTNYGVHWVRQSPGKGLE
WLGVIWSGGNTNYNTPFTSRLSINKDNSKSQVFFKMNSLQSNDTAIYYCARALTYYNYQFAYWGQ
GTLVTVSAASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELLGGPS

VFLFPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCSVMHEALHN
HYTQKSLSLSPGK

## Claims

1. An epidermal growth factor receptor (EGFR) antibody that binds preferentially to disease cells having an EGFR density greater than a normal EGFR density, wherein the disease cells present with more than 10,000 EGFR molecules per cell and the normal cells present with not more than 10,000 EGFR molecules per cell, the EGFR antibody comprising a heavy chain and a light chain, each chain having a constant region and a variable region, each variable region comprising framework region sequences of cetuximab and complementarity determining regions (CDRs), wherein the CDRs have an amino acid sequence set forth below:
For the heavy chain:
| | | |
|---|---|---|
| CDR1 | NYGVH | (SEQ ID No. 1) |
| CDR2 | VIWSGGNTD⁵⁸YNTPFTS | (SEQ ID No. 2) |
| CDR3 | ALTY¹⁰¹Y¹⁰²D¹⁰³YE¹⁰⁵FAY | (SEQ ID No. 3) |
For the light chain:
| | | |
|---|---|---|
| CDR1 | RASQSIGTNIH | (SEQ ID No. 4) |
| CDR2 | ASE⁵³SIS | (SEQ ID No. 5) |
| CDR3 | QQNNNW⁹⁴PTT | (SEQ ID No. 6) |
wherein at least one of E⁵³ is substituted by K⁵³; D⁵⁸ is substituted by N⁵⁸; W⁹⁴ is substituted by A⁹⁴; Y¹⁰¹ is substituted by A¹⁰¹; Y¹⁰² is substituted by A¹⁰²; D¹⁰³ is substituted by N¹⁰³; E¹⁰⁵ is substituted by Q¹⁰⁵, wherein the substituted amino acid confers on said antibody a reduced EGFR binding affinity (Kd) that is 1.0 nM or weaker, wherein when D⁵⁸ is substituted by N⁵⁸ this is in combination with D¹⁰³ is substituted by N¹⁰³ and when E¹⁰⁵ is substituted by Q¹⁰⁵ this is in combination with D⁵⁸ being substituted by N⁵⁸ and D¹⁰³ being substituted by N¹⁰³.

2. The antibody according to claim 1, wherein Y¹⁰¹ is substituted by A¹⁰¹, Y¹⁰² is substituted by A¹⁰² or D¹⁰³ is substituted by N¹⁰³.

3. The antibody according to claim 1 or 2, wherein E⁵³ and Y¹⁰² are both substituted.

4. The antibody according to any one of claims 1 to 3, wherein E⁵³ is substituted by K⁵³ and Y¹⁰² is substituted by A¹⁰².

5. The antibody according to claim 1, wherein E⁵³ is substituted by K⁵³.

6. The antibody according to claim 1, wherein W⁹⁴ is substituted by A⁹⁴.

7. An antibody according to any preceding claim, the antibody having the constant region sequence of cetuximab.

8. An antibody according to claim 1, which is [D⁵⁸N,D¹⁰³N]cetuximab.

9. A bivalent fragment of an antibody according to any one of claims 1-8 that binds preferentially to disease cells having an EGFR density greater than a normal EGFR density.

10. A conjugate comprising a cytotoxin or a detectable label and, conjugated thereto, an antibody or bivalent fragment thereof as defined according to any one of claims 1-9.

11. A pharmaceutical composition comprising a pharmaceutically acceptable carrier and an EGFR antibody, bivalent fragment thereof and/or conjugate according to any one of claims 1 to 10.

12. The pharmaceutical composition of claim 11 or an EGFR antibody according to any one of claims 1 to 8, a bivalent fragment according to claim 9 and/or a conjugate according to claim 10 for use as a medicament.

13. The pharmaceutical composition of claim 11 or an EGFR antibody according to any one of claims 1 to 8, a bivalent fragment according to claim 9 and/or a conjugate according to claim 10 for use in the treatment of cancer.

14. Composition for use according to claim 13 wherein said cancer is EGFR-presenting cancers that include head and neck cancers, especially squamous cell carcinoma of the head and neck, colorectal cancers, gastrointestinal cancers, brain tumours including glioblastomas, and tumours of the lung including non-small-cell lung carcinoma, and of the breast, pancreas, esophagus, kidney, ovary, cervix and prostate.

## Patentansprüche

1. Epidermal Growth Factor-Rezeptor (EGFR)-Antikörper, der bevorzugt an Krankheitszellen mit einer EGFR-Dichte bindet, die größer ist als eine normale EGFR-Dichte, wobei die Krankheitszellen mehr als 10.000 EGFR-Moleküle pro Zelle zeigen und die normalen Zellen nicht mehr als 10.000 EGFR-Moleküle pro Zelle zeigen, wobei der EGFR-Antikörper eine schwere Kette und eine leichte Kette umfasst, wobei jede Kette eine konstante Region und eine variable Region aufweist, wobei jede variable Region Gerüstregionssequenzen von Cetuximab und komplementaritätsbestimmende Regionen (CDRs) aufweist, wobei die CDRs eine Aminosäuresequenz aufweisen, wie nachstehend angegeben:
Für die schwere Kette:
| | | |
|---|---|---|
| CDR1 | NYGVH | (SEQ ID No. 1) |
| CDR2 | VIWSGGNTD⁵⁸YNTPFTS | (SEQ ID No. 2) |
| CDR3 | ALTY¹⁰¹Y¹⁰²D¹⁰³YE¹⁰⁵FAY | (SEQ ID No. 3) |
Für die leichte Kette:
| | | |
|---|---|---|
| CDR1 | RASQSIGTNIH | (SEQ ID No. 4) |
| CDR2 | ASE⁵³SIS | (SEQ ID No. 5) |
| CDR3 | QQNNNW⁹⁴PTT | (SEQ ID No. 6), |
wobei wenigstens eines von E⁵³ durch K⁵³ ersetzt ist, D⁵⁸ durch N⁵⁸ ersetzt ist, W⁹⁴ durch A⁹⁴ ersetzt ist, Y¹⁰¹ durch A¹⁰¹ ersetzt ist, Y¹⁰² durch A¹⁰² ersetzt ist, D¹⁰³ durch N¹⁰³ ersetzt ist, E¹⁰⁵ durch Q¹⁰⁵ ersetzt ist, wobei die ausgetauschte Aminosäure dem Antikörper eine verringerte EGFR-Bindungsaffinität (Kd) verleiht, die 1,0 nM oder schwächer ist, wobei, wenn D⁵⁸ durch N⁵⁸ ersetzt ist, dies damit kombiniert ist, dass D¹⁰³ durch N¹⁰³ ersetzt ist, und wenn E¹⁰⁵ durch Q¹⁰⁵ ersetzt ist, dies damit kombiniert ist, dass D⁵⁸ durch N⁵⁸ ersetzt ist und D¹⁰³ durch N¹⁰³ ersetzt ist.

2. Antikörper nach Anspruch 1, wobei Y¹⁰¹ durch A¹⁰¹ ersetzt ist, Y¹⁰² durch A¹⁰² ersetzt ist oder D¹⁰³ durch N¹⁰³ ersetzt ist.

3. Antikörper nach Anspruch 1 oder 2, wobei E⁵³ und Y¹⁰² beide ersetzt sind.

4. Antikörper nach einem der Ansprüche 1 bis 3, wobei E⁵³ durch K⁵³ ersetzt ist und Y¹⁰² durch A¹⁰² ersetzt ist.

5. Antikörper nach Anspruch 1, wobei E⁵³ durch K⁵³ ersetzt ist.

6. Antikörper nach Anspruch 1, wobei W⁹⁴ durch A⁹⁴ ersetzt ist.

7. Antikörper nach einem der vorhergehenden Ansprüche, wobei der Antikörper die Konstantregionssequenz von Cetuximab besitzt.

8. Antikörper nach Anspruch 1, nämlich [D⁵⁸N,D¹⁰³N]-Cetuximab.

9. Bivalentes Fragment eines Antikörpers nach einem der Ansprüche 1 bis 8, das bevorzugt an Krankheitszellen mit einer EGFR-Dichte bindet, die größer ist als eine normale EGFR-Dichte.

10. Konjugat, umfassend ein Zytotoxin oder eine nachweisbare Markierung und, damit konjugiert, einen Antikörper oder ein bivalentes Fragment davon, wie in einem der Ansprüche 1 bis 9 definiert.

11. Pharmazeutische Zusammensetzung, umfassend einen pharmazeutisch annehmbaren Träger und einen EGFR-Antikörper, ein bivalentes Fragment davon und/oder ein Konjugat nach einem der Ansprüche 1 bis 10.

12. Pharmazeutische Zusammensetzung nach Anspruch 11 oder EGFR-Antikörper nach einem der Ansprüche 1 bis 8, bivalentes Fragment nach Anspruch 9 und/oder Konjugat nach Anspruch 10 zur Verwendung als Arzneimittel.

13. Pharmazeutische Zusammensetzung nach Anspruch 11 oder EGFR-Antikörper nach einem der Ansprüche 1 bis 8, bivalentes Fragment nach Anspruch 9 und/oder Konjugat nach Anspruch 10 zur Verwendung bei der Behandlung von Krebs.

14. Zusammensetzung zur Verwendung nach Anspruch 13, wobei der Krebs ein EGFR-involvierender Krebs ist, einschließlich Kopf-Halskrebs, insbesondere Plattenepithelkarzinom des Kopfes und Nackens, Kolorektalkrebs, Gastrointestinalkrebs, Hirntumoren, einschließlich Glioblastomen, und Tumoren der Lunge, einschließlich nicht-kleinzelligen Lungenkarzinoms, und der Brust, des Pankreas, Ösophagus, der Niere, Eierstöcke, Zervix und Prostata.

## Revendications

1. Anticorps anti-récepteur du facteur de croissance épidermique (EGFR) qui se lie préférentiellement à des cellules pathologiques présentant une densité d'EGFR supérieure à une densité d'EGFR normale, lesdites cellules pathologiques se présentant avec plus de 10 000 molécules d'EGFR par cellule et les cellules normales se présentant avec pas plus de 10 000 molécules d'EGFR par cellule, ledit anticorps anti-EGFR comprenant une chaîne lourde et une chaîne légère, chaque chaîne comportant une région constante et une région variable, chaque région variable comprenant des séquences de régions charpentes de cetuximab et des régions déterminant la complémentarité (CDR), lesdites CDR comportant une séquence d'acides aminés indiquée ci-dessous :
Pour la chaîne lourde :
| | | |
|---|---|---|
| CDR1 | NYGVH | (SEQ ID n° : 1) |
| CDR2 | VIWSGGNTD⁵⁸YNTPFTS | (SEQ ID n° : 2) |
| CDR3 | ALTY¹⁰¹Y¹⁰²D¹⁰³YE¹⁰⁵FAY | (SEQ ID n° : 3) |
Pour la chaîne légère :
| | | |
|---|---|---|
| CDR1 | RASQSIGTNIH | (SEQ ID n° : 4) |
| CDR2 | ASE⁵³SIS | (SEQ ID n° : 5) |
| CDR3 | QQNNNW⁹⁴PTT | (SEQ ID n° : 6) |
dans lesquelles au moins l'un de E⁵³ est substitué par K⁵³ ; D⁵⁸ est substitué par N⁵⁸ ; W⁹⁴ est substitué par A⁹⁴ ; Y¹⁰¹ est substitué par A¹⁰¹ ; Y¹⁰² est substitué par A¹⁰² ; D¹⁰³ est substitué par N¹⁰³ ; E¹⁰⁵ est substitué par Q 10⁵ ; l'acide aminé substitué conférant audit anticorps une affinité de liaison à l'EGFR réduite (Kd) qui est de 1,0 nM ou plus faible ; lorsque D⁵⁸ est substitué par N⁵⁸, celui-ci étant en combinaison avec D¹⁰³ qui est substitué par N¹⁰³, et lorsque E¹⁰⁵ est substitué par Q¹⁰⁵, celui-ci étant en combinaison avec D⁵⁸ qui est substitué par N⁵⁸ et D¹⁰³ qui est substitué par N¹⁰³.

2. Anticorps selon la revendication 1, Y¹⁰¹ étant substitué par A¹⁰¹, Y¹⁰² étant substitué par A¹⁰² ou D¹⁰³ étant substitué par N¹⁰³.

3. Anticorps selon la revendication 1 ou 2, E⁵³ et Y¹⁰² étant tous deux substitués.

4. Anticorps selon l'une quelconque des revendications 1 à 3, E⁵³ étant substitué par K⁵³ et Y¹⁰² étant substitué par A¹⁰².

5. Anticorps selon la revendication 1, E⁵³ étant substitué par K⁵³.

6. Anticorps selon la revendication 1, W⁹⁴ étant substitué par A⁹⁴.

7. Anticorps selon l'une quelconque des revendications précédentes, l'anticorps comportant la séquence de région constante du cetuximab.

8. Anticorps selon la revendication 1, qui est le [D⁵⁸N, D¹⁰³N]cetuximab.

9. Fragment bivalent d'un anticorps selon l'une quelconque des revendications 1 à 8 qui se lie préférentiellement à des cellules pathologiques présentant une densité d'EGFR supérieure à une densité d'EGFR normale.

10. Conjugué comprenant une cytotoxine ou un marqueur détectable et, conjugué à celui-ci, un anticorps ou un fragment bivalent de celui-ci tel que défini selon l'une quelconque des revendications 1 à 9.

11. Composition pharmaceutique comprenant un vecteur pharmaceutiquement acceptable et un anticorps anti-EGFR, un fragment bivalent de celui-ci et/ou un conjugué selon l'une quelconque des revendications 1 à 10.

12. Composition pharmaceutique selon la revendication 11 ou anticorps anti-EGFR selon l'une quelconque des revendications 1 à 8, fragment bivalent selon la revendication 9 et/ou conjugué selon la revendication 10 pour une utilisation en tant que médicament.

13. Composition pharmaceutique selon la revendication 11 ou anticorps anti-EGFR selon l'une quelconque des revendications 1 à 8, fragment bivalent selon la revendication 9 et/ou conjugué selon la revendication 10 pour une utilisation dans le traitement d'un cancer.

14. Composition pour une utilisation selon la revendication 13, ledit cancer étant des cancers présentant l'EGFR qui comprennent les cancers de la tête et du cou, en particulier le carcinome épidermoïde de la tête et du cou, les cancers colorectaux, les cancers gastro-intestinaux, les tumeurs cérébrales dont les glioblastomes, et les tumeurs du poumon, dont le carcinome pulmonaire non à petites cellules, et du sein, du pancréas, de l'œsophage, des reins, des ovaires, du col de l'utérus et de la prostate.
